(19) Europäisches Patentamt
European Patent Office
Office européen des brevets

(11) EP 0 901 486 B1

(12) **FASCICULE DE BREVET EUROPEEN**

(45) Date de publication et mention de la délivrance du brevet:
**26.09.2001 Bulletin 2001/39**

(21) Numéro de dépôt: **98908160.9**

(22) Date de dépôt: **10.02.1998**

(51) Int Cl.[7]: **C07D 339/00**, C07D 337/02, C07D 337/16, C07D 339/04, C07D 339/08, C07D 341/00, C07D 339/06, C08F 220/38, G02B 1/04

(86) Numéro de dépôt international:
**PCT/FR98/00254**

(87) Numéro de publication internationale:
**WO 98/35955 (20.08.1998 Gazette 1998/33)**

(54) **NOUVEAUX MONOMERES (THIO)(METH)ACRYLATES, COMPOSES INTERMEDIAIRES POUR LA SYNTHESE DE CES MONOMERES, COMPOSITIONS POLYMERISABLES ET POLYMERES OBTENUS ET LEURS APPLICATIONS OPTIQUES ET OPHTALMIQUES**

NEUE (THIO)(METH)ACRYLAT-MONOMERE, ZWISCHENPRODUKTE ZU IHRER HERSTELLUNG, POLYMERISIERBARE ZUSAMMENSETZUNGEN UND DIE DARAUS RESULTIERENDEN POLYMERE UND DEREN OPTISCHE UND AUGENHEILKUNDLICHE VERWENDUNG

NOVEL (THIO)(METH)ACRYLATE MONOMERS, INTERMEDIATE COMPOUNDS FOR THE SYNTHESIS OF THESE MONOMERS, POLYMERISABLE COMPOSITIONS AND RESULTING POLYMERS AND OPTICAL AND OPHTHALMOLOGIC APPLICATIONS THEREOF

(84) Etats contractants désignés:
**DE ES FR GB IT**

(30) Priorité: **13.02.1997 FR 9701687**

(43) Date de publication de la demande:
**17.03.1999 Bulletin 1999/11**

(73) Titulaire: **ESSILOR INTERNATIONAL COMPAGNIE GENERALE D'OPTIQUE**
**94227 Charenton cédex (FR)**

(72) Inventeurs:
- **CAYE, Florence**
  **F-57500 Saint Avold (FR)**
- **SINDT, Michèle**
  **F-57420 Verny (FR)**
- **PAQUER, Daniel**
  **F-54500 Vandoeuvre (FR)**
- **JURY, Dorothée**
  **F-38121 Chonas-l'Amballan (FR)**
- **SCHNEIDER, Michel**
  **F-57050 Le Ban-Saint-Martin (FR)**
- **MIELOSZYNSKI, Jean-Luc**
  **F-57130 Ars-sur-Moselle (FR)**

(74) Mandataire: **Catherine, Alain et al**
**Cabinet Harlé & Phélip**
**7, rue de Madrid**
**75008 Paris (FR)**

(56) Documents cités:
**EP-A- 0 435 305** **EP-A- 0 728 572**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 901 486 B1

## Description

La présente invention concerne, d'une manière générale, de nouveaux monomères (thio)(méth)acrylates, de préférence mono(thio) (méth)acrylates, utiles pour la formulation de compositions polymérisables conduisant à des homopolymères et copolymères transparents appropriés pour des applications optiques et ophtalmiques.

Les compositions polymérisables, selon l'invention, permettent la fabrication d'objets moulés en polymère transparent, de préférence thermoplastique, par polymérisation dans des moules ou par moulage par injection.

Les polymères transparents obtenus, qui ont des indices de réfraction de valeur moyenne à élevée, 1,54 ou plus, conviennent particulièrement pour des applications optiques et ophtalmiques.

Parmi les applications optiques des polymères, selon l'invention, on peut citer les guides d'onde et les fibres optiques.

Parmi les applications ophtalmiques de ces polymères, on peut citer les verres de lunettes et les lentilles de contact.

De manière générale, les nouveaux monomères, selon la présente invention, sont des monomères fonctionnels de type mono(thio) (méth)acrylates ou mono- et di- (méth)acrylates porteurs d'un hétérocycle constitué d'atomes d'hydrogène, carbone et soufre, ayant 5 à 8 chaînons et au moins deux atomes de soufre intracycliques. De préférence, l'hétérocycle comporte 6 ou 7 chaînons, mieux 6 chaînons. De préférence, également, le nombre d'atomes de soufre intracycliques est 2 ou 3. L'hétérocycle peut éventuellement être condensé avec un cycle aromatique ou polycyclanique en $C_5$-$C_8$, substitué ou non, de préférence un cycle en $C_6$-$C_7$.

Lorsque l'hétérocyclique des monomères fonctionnels selon l'invention comporte 2 atomes de soufre intracycliques, de préférence ces atomes de soufre intracycliques sont en position 1-3 ou 1-4 de l'hétérocycle. Le monomère est également de préférence, selon l'invention, un monomère thio(méth)acrylate. Enfin, les monomères selon l'invention ont des masses molaires de préférence comprises entre 150 et 400, de préférence 150 et 350, et mieux encore, entre 200 et 300.

Plus particulièrement, les nouveaux monomères fonctionnels selon l'invention répondent à la formule :

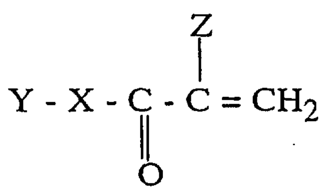

(A)

dans laquelle Z représente H ou $CH_3$ (préferentiellement $CH_3$) et X représente O ou S, et

- lorsque X représente S, Y représente un radical de formule :

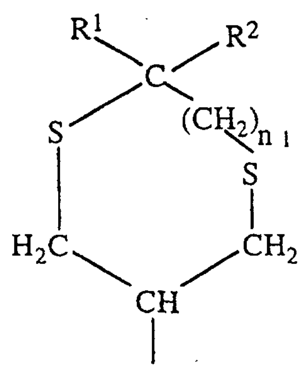

(a)

  où $R^1$ et $R^2$ sont choisis parmi H, les radicaux alkyle, de préférence les radicaux alkyle en $C_1$-$C_4$, et mieux le radical $CH_3$, ou encore $R^1$ et $R^2$ forment conjointement un radical $(CH_2)_5$, et $n_1$ est un entier de 0 à 2 inclus, et
- lorsque X représente O, Y représente le radical (a) défini ci-dessus ou un radical choisi parmi les radicaux de formules :

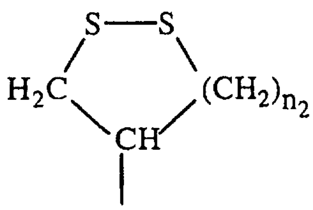 (b) , 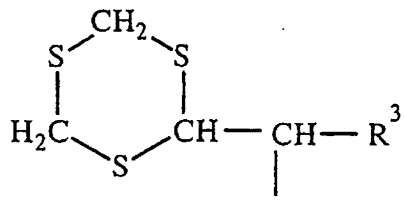 (c)

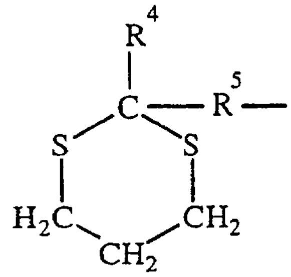 (d) et 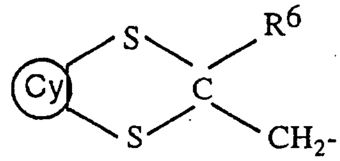 (e)

dans lesquelles $n_2$ est égal à 1 ou 2, $R^3$ représente H ou un radical alkyle, de préférence un radical alkyle en $C_1$-$C_4$ et mieux le radical $CH_3$, $R^4$ représente H ou un radical alkyle, de préférence un radical alkyle en $C_1$-$C_4$, et mieux le radical $CH_3$ ou $C_2H_5$, et $R^5$ est un radical divalent choisi parmi les groupements de formules suivantes :

(I) 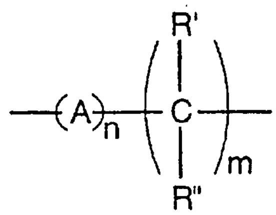

dans laquelle :

A désigne un groupe aryle, préférentiellement en $C_6$-$C_{12}$, et mieux un groupe phényle ou un groupe alkyle préférentiellement en $C_1$-$C_6$,

R', R" désignent indépendamment l'un de l'autre H, un groupe alkyle, préférentiellement un groupe alkyle en $C_1$-$C_6$, aryle, préférentiellement phényle, ou l'un de R', R" peut être un groupe

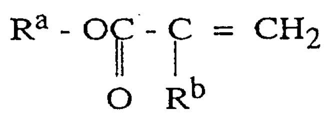

où $R^a$ est un groupe alkylène, de préférence en $C_1$-$C_6$, en particulier un groupe $-CH_2$ -, et $R^b$ est H ou $CH_3$, n prend les valeurs O ou 1 et O ≤ m (entier ≤ 4), et

(II) 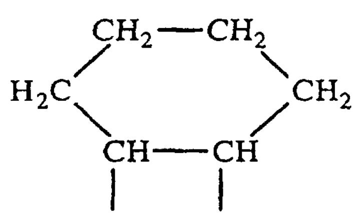 ,

et
$R_6$ désigne H ou $CH_3$ , et
Cy désigne un noyau aryle substitué ou non, de préférence un noyau phényle, tolyle ou norbornyle.

De préférence, $R^5$ est un radical divalent choisi parmi :

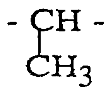 , 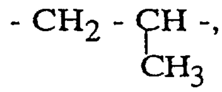 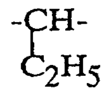

et

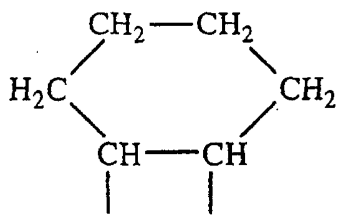

Les monomères selon l'invention, particulièrement recommandés, sont les monomères thio(méth)acrylates répondant à la formule (A) ci-dessus dans laquelle X représente un atome de soufre et Y est un radical de formule (a).
Les monomères, selon l'invention, peuvent être préparés par divers procédés de synthèse connus.
Parmi les monomères mono(thio)(méth)acryliques préférés, selon l'invention, on peut citer les monomères de formules :

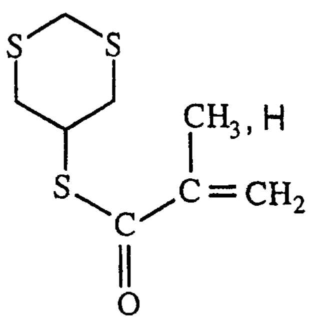 ,

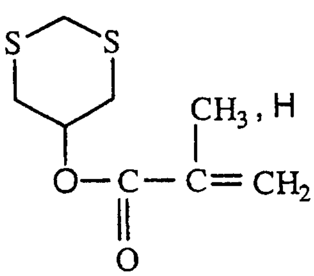 , 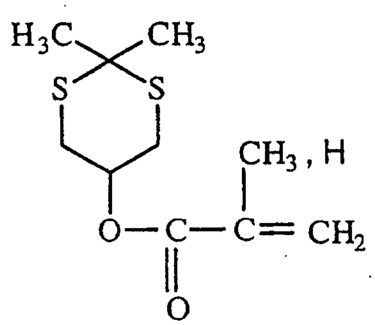 ,

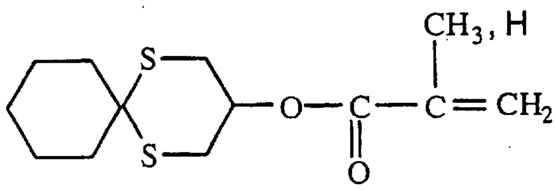 ,

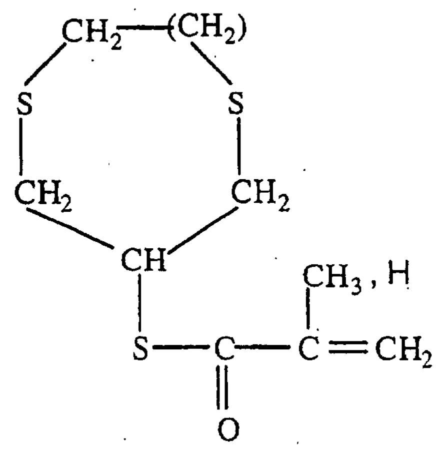 ,

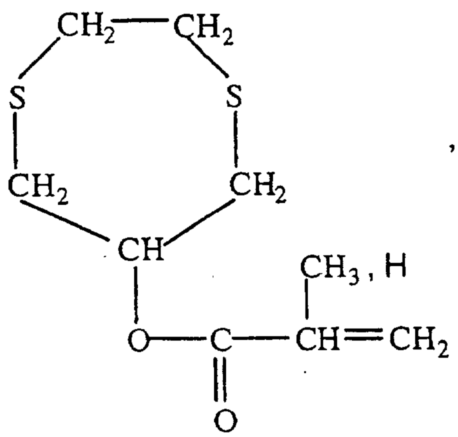 ,

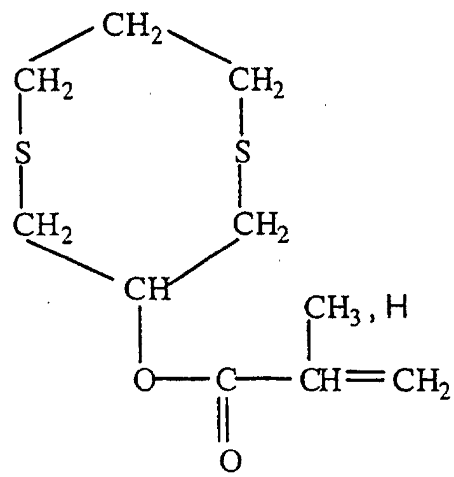 ,

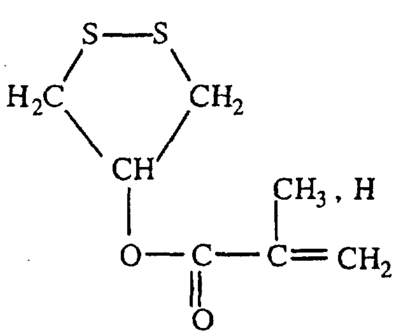 ,

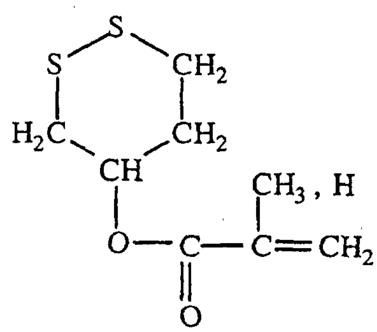 ,

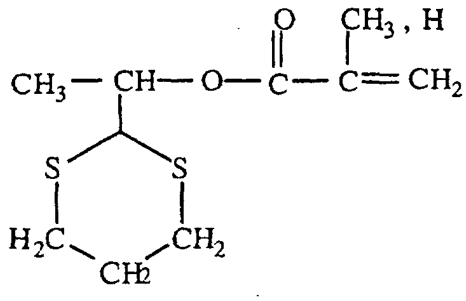 , 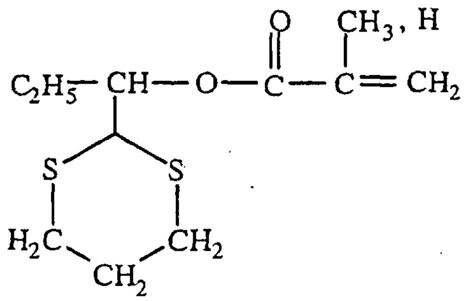 ,

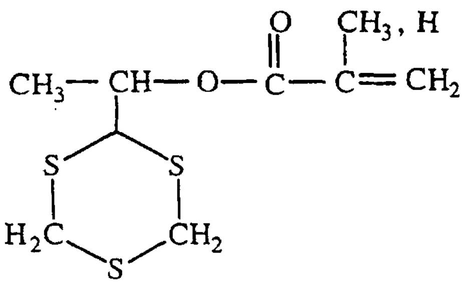 , 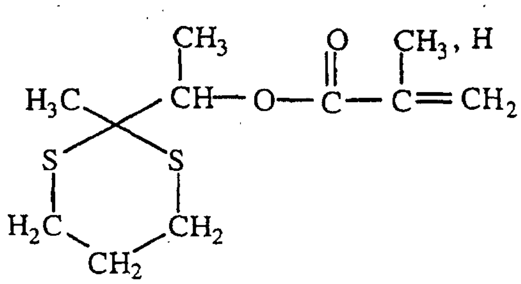 ,

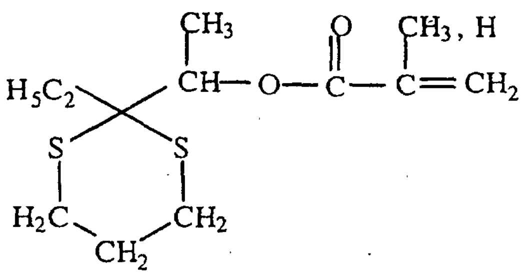 , 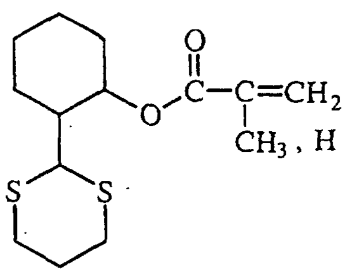 ,

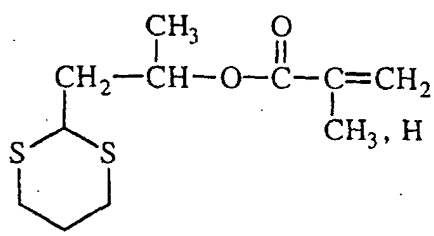

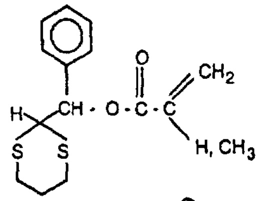 ,

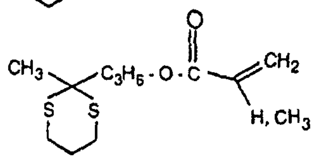 ,

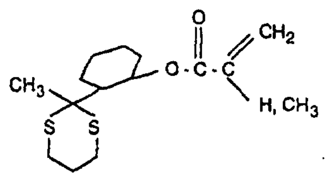 ,

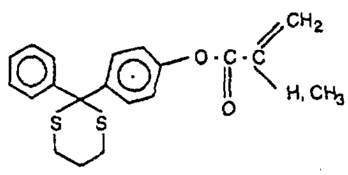 ,

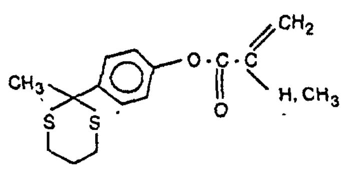 ,

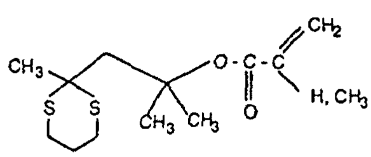 ,

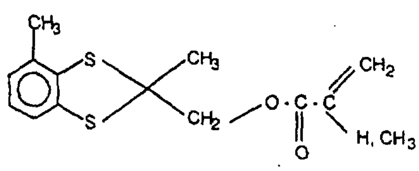 , et

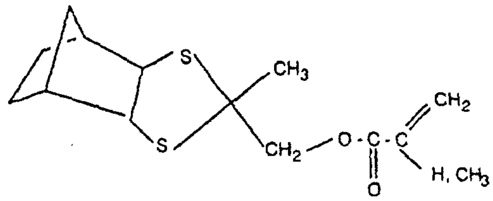

Parmi les monomères di(méth)acryliques préférés selon l'invention, on peut citer les monomères de formules :

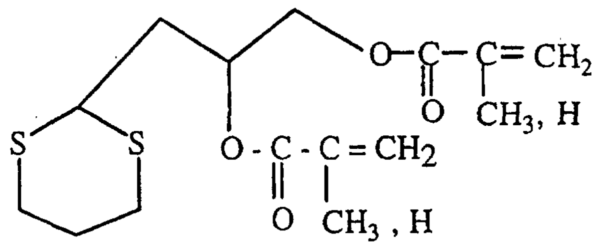

,

et en particulier le diméthacrylate.

La suite de la description donne différents exemples de synthèse des monomères selon l'invention.

## I. Synthèse de cycles soufrés par réaction de Dieckmann

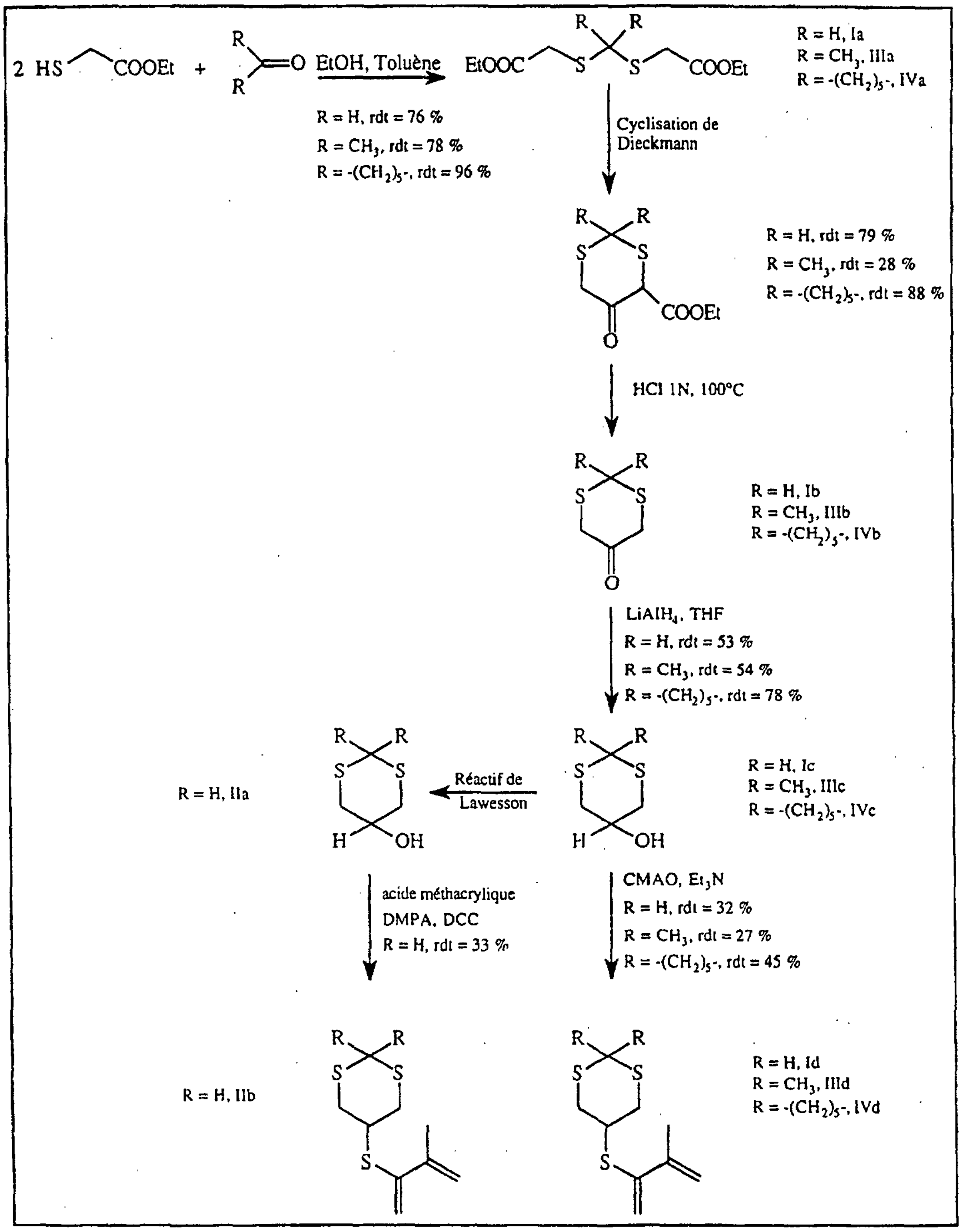


CMAO = chlorure de méthacryloyle.

DMAP = diméthylaminopyridine.

DCC = dicyclohexylcarbodiimide.

**I.1 Synthèse des diesters Ia, IIIa, IVa**

Dans un tricol de 250 ml, équipé d'un réfrigérant, on introduit 0,2 mole de mercaptoacétate d'éthyle (2 éq.) dans 30 ml de toluène, et 1 ml d'acide sulfurique concentré.

Le mélange réactionnel est porté à une température de 80°C, puis on additionne goutte à goutte, soit 0,1 mole de formaldéhyde en solution à 36% dans l'eau (stabilisé au méthanol) dans le cas de la préparation du composé **Ia,** soit 0,1 mole d'acétone ou cyclohexanone dans le cas des composés **IIIa** et **IVa.**

A l'issue de cette addition, on porte le mélange réactionnel à 100°C pendant 2 heures. Puis l'agitation est maintenue pendant une nuit à température ambiante.

Le mélange réactionnel est concentré sous pression réduite. Le résidu est repris avec du dichlorométhane, lavé successivement avec une solution d'hydroxyde de sodium à 5% puis avec de l'eau.

La phase organique est séchée sur sulfate de sodium, filtrée puis concentrée sous pression réduite.

Le produit ainsi obtenu est purifié par distillation sous pression réduite.

**Ia : diéthyl 3,5-dithiaheptane 1,7-dicarboxylate**

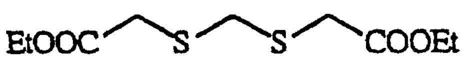

Rdt = 76%.
Point d'ébullition = 126°C/(0,1 mmHg) 13,33 Pa.

**IIIa : diéthyl 3,5-dithia(4-diméthyl)heptane 1,7-dicarboxylate**

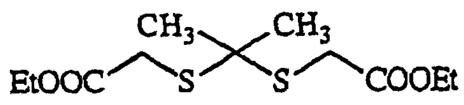

Rdt = 78%.
Point d'ébullition = 131°C (0,1mmHg)/13.33 Pa.

**IVa : diéthyl 3,5-dithia(4-cyclohexyl)heptane 1,7-dicarboxylate**

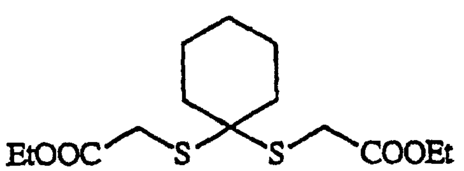

Rdt=96%.
Point d'ébullition = 165°C/ (0,3 mmHg) 39,99 Pa.

**I.2 Cyclisation de Dieckmann : utilisation de méthylate de sodium dans l'éther**

0,1 mole (2 éq.) de méthylate de sodium fraîchement préparé est mise en suspension dans 70 ml d'éther anhydre; à température ambiante, on additionne goutte à goutte 0,05 mole (1 éq.) de diester dissout dans 20 ml d'éther anhydre. Puis le mélange réactionnel est agité pendant dix heures à reflux. On laisse revenir à température ambiante, puis on verse le mélange réactionnel dans un mélange eau-glace-acide acétique.

La phase aqueuse est extraite avec deux fois 60 ml d'éther. Les phases éthérées sont lavées avec une solution diluée d'hydrogénocarbonate de sodium puis à l'eau.

La phase organique est séchée sur sulfate de sodium, et concentrée sous pression réduite.
Les β-cétoesters ainsi obtenus sont utilisés brut dans la suite de la synthèse.

**I.3 Décarboxylation de β-cétoesters en milieu acide chlorhydrique.**

Dans un ballon équipé d'un réfrigérant, on introduit 0,05 mole de β-cétoester et 120 ml d'une solution d'acide chlorhydrique IN. On porte le mélange réactionnel à 100°C pendant 20 heures minimum. Le mélange réactionnel est repris avec 100 ml d'acétate d'éthyle. La phase aqueuse est neutralisée avec une solution d'hydroxyde de sodium jusqu'à pH 5.
La phase organique est ensuite lavée successivement à l'eau puis avec une solution saturée de chlorure de sodium.
La phase organique est séchée sur sulfate de sodium puis concentrée sous pression réduite.
Les thiacycloalcan 3-ones ainsi obtenues seront purifiées par chromatographie sur gel de silice.

**Ib : 1,3-dithiacyclohexan 5-one**

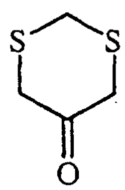

Rdt = 79% concerne les deux étapes précédentes.
Point de fusion = 101°C.

**IIb : 2-diméthyl 1,3-dithiacyclohexan 5-one**

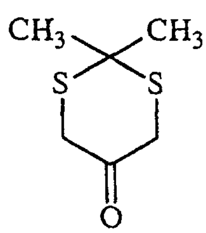

Rdt = 28% concerne les deux étapes précédentes.
Eluant de purification : 9% acétate d'éthyle/91% éther de pétrole.

**IV b : 2-cyclohexyl 1,3-dithiacyclohexan 5-one**

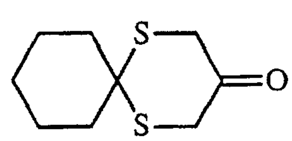

Rdt = 88% concerne les deux étapes précédentes.
Eluant de purification : 10% acétate d'éthyle/90% éther de pétrole.

**I.4 Réduction d'une cétone par LiAlH4 dans THF**

Dans un tricol muni d'un réfrigérant, d'une agitation et d'une ampoule à brome, on introduit 0,05 mole (1 éq.) d'hydrure de lithium et d'aluminium en suspension dans 20 ml de tétrahydrofuranne anhydre sous atmosphère d'azote.

On ajoute alors goutte à goutte 0,05 mole (1 éq.) de la cétone obtenue à l'étape I.3, dissoute dans 10 ml de tétrahydrofuranne, à température ambiante.

Après addition, on porte le mélange réactionnel à reflux pendant 12 heures. Puis on laisse la solution revenir à température ambiante. Le mélange réactionnel est refroidi à 0°C pour être hydrolysé avec 20 ml d'eau.

La solution est alors versée dans 60 ml d'une solution d'acide sulfurique à 10%. La phase organique est séparée, et la phase aqueuse est extraite avec trois fois 60 ml d'éther. Les phases éthérées combinées, sont lavées successivement avec 50 ml d'eau et 50 ml d'une solution saturée d'hydrogénocarbonate de sodium, puis séchées sur sulfate de sodium et concentrées sous pression réduite.

**Ic : 1,3-dithiacyclohexan 5-ol**

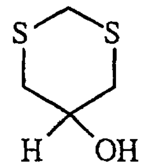

Rdt = 53%.
Eluant de purification : 20% acétate d'éthyle/80% éther de pétrole.

**IIIc : 2-diméthyl 1,3-dithiacyclohexan 5-ol**

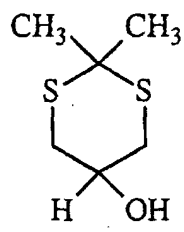

Rdt = 54%.
Eluant de purification : % acétate d'éthyle/% éther de pétrole.

**IVc : 2-cyclohexyl 1,3-dithiacyclohexan 5-ol**

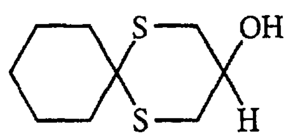

Rdt = 78%.
Eluant de purification : % acétate d'éthyle/% éther de pétrole.

**I.5 Synthèse de mercaptans à partir d'alcools : utilisation du réactif de Lawesson**

Sous atmosphère d'azote, on introduit 20 mmoles (1 éq.) d'alcool obtenu à l'étape précédente, dissous dans 60 ml de toluène ainsi que 11 mmoles (0,55 éq.) de réactif de Lawesson.

Le mélange réactionnel est porté à reflux pendant un temps variable selon le substrat.

Le suivi réactionnel s'effectue par chromatographie sur couche mince.

Après disparition de l'alcool, le mélange réactionnel est repris avec 100 ml d'eau et extrait avec deux fois 50 ml de dichlorométhane.

Les phases organiques combinées sont séchées sur sulfate de sodium, puis concentrées sous pression réduite.

Les mercaptans ainsi obtenus sont utilisés bruts dans la suite de la synthèse.

**IIa : 1,3-dithiacyclohexan 5-thiol**

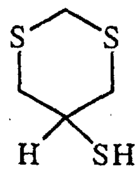

**I.6 Condensation du CMAO sur un alcool**

Dans un tricol muni d'un réfrigérant, d'un thermomètre et d'une agitation magnétique, on introduit 0,05 mole (1 éq.) d'alcool obtenu à l'étape I.4, 0,05 mole (1 éq.) de triéthylamine et 800 ppm d'éther monométhylique de l'hydroquinone (EMHQ) dans 300 ml de chloroforme. Le mélange réactionnel est refroidi à 0°C; puis on additionne goutte à goutte 0,055 mole (1 éq.) de chlorure de méthacryloyle en solution dans 20 ml de chloroforme, tout en maintenant la température à 0°C.

Après retour à température ambiante, on laisse agiter pendant 48 heures.

On acidifie le mélange réactionnel avec 50 ml d'une solution d'acide sulfurique 6N, et on extrait à l'éther. La phase organique est lavée successivement avec une solution d'hydrogénocarbonate de sodium à 10% puis avec une solution saturée en chlorure de sodium.

La phase éthérée est séchée sur sulfate de sodium et le solvant est évaporé.

Les composés méthacryliques ainsi obtenus sont purifiés par chromatographie sur gel de silice.

**Id**

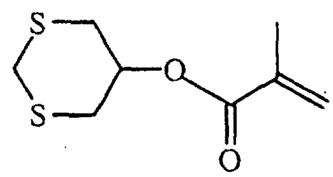

Rdt = 32%.
Eluant de purification : 1% acétate d'éthyle/99% éther de pétrole.

**IIId**

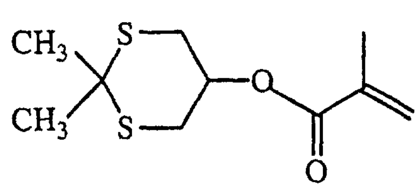

Rdt = 27%.
Eluant de purification : 2% acétate d'éthyle/98% éther de pétrole.

**IVd**

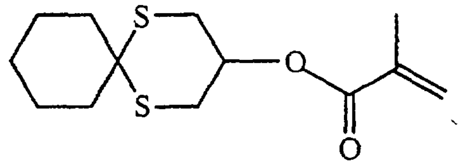

Rdt = 45%.
Eluant de purification : 1% acétate d'éthyle/99% éther de pétrole.

**I.7 Obtention de thioester méthacrylique par réaction d'un mercaptan sur l'acide méthacrylique en présence de dicyclo-hexylecarbodiimide et diméthylaminopyridine.**

Dans un tricol de 250 ml, on introduit 65 mmoles d'acide méthacrylique et 800 ppm d'EMHQ dans 70 ml de dichlorométhane sous agitation. A température ambiante, on additionne goutte à goutte 32,5 mmoles de mercaptan obtenu à l'étape I.5 et 650 mg de diméthylaminopyridine (DMAP) (quantité catalytique 5% en masse par rapport à l'acide). Le mélange réactionnel est alors refroidi à 0°C et on ajoute 65 mmoles de dicyclohexylcarbodiimide (DCC). L'agitation est maintenue à 0°C pendant cinq minutes, puis pendant cinq heures à température ambiante.
Le mélange réactionnel est filtré pour éliminer la dicyclohexylurée formée.
Le filtrat est repris avec du dichlorométhane, la phase organique est lavée successivement avec une solution 0,5 N d'acide chlorhydrique puis avec une solution 0,5 N d'hydroxyde de sodium. La phase organique est séchée, filtrée et concentrée sous pression réduite.
Le thioester méthacrylique ainsi obtenu est purifié par chromatographie sur gel de silice.

**IIb**

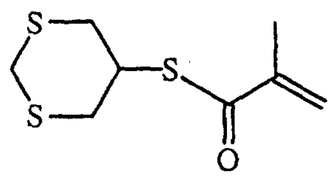

Rdt = 33%.
Eluant de purification : 2% acétate d'éthyle/98% éther de pétrole.

**II. Préparation de cycles soufrés par réaction de cyclisation intermoléculaire**

Cette méthode de cyclisation permet d'aboutir à une série de monomères méthacryliques de dithiacycloalcanes selon le schéma ci-dessous.
Les différentes étapes de cette synthèse sont :

- la condensation d'un dimercaptan sur la dichloroacétone,
- la réduction par l'hydrure de lithium et d'aluminium dans le tétrahydrofuranne, et
- la condensation du chlorure de méthacryloyle sur l'alcool précédemment obtenu.

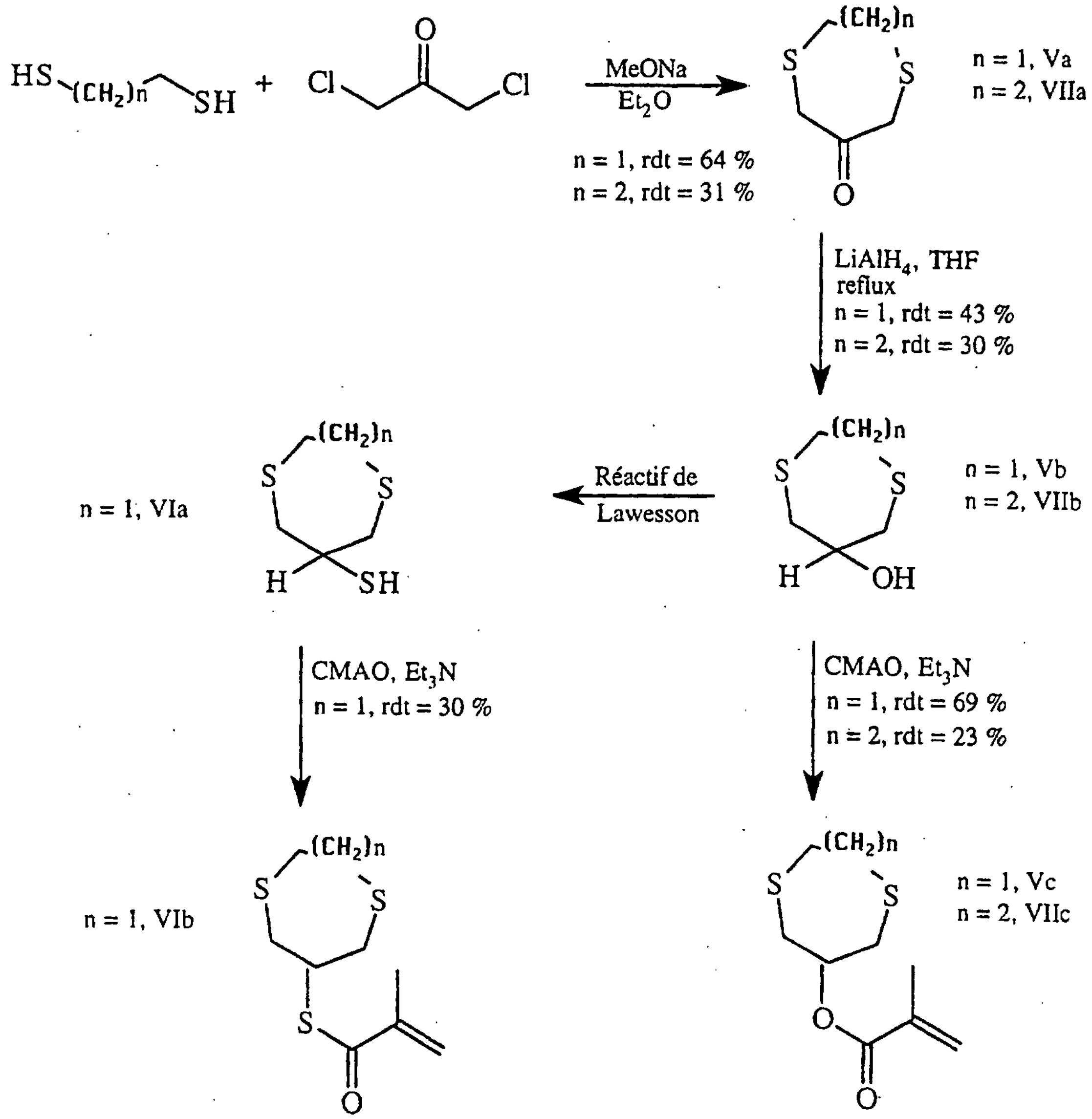


**MODES OPERATOIRES**

**II.1 Condensation de la dichloroacétone sur un dimercaptan**

0,1 mole (2 éq.) de méthylate de sodium fraîchement préparé est dissoute dans 20 ml de méthanol anhydre sous agitation, puis on additionne à température ambiante 0,05 mole (1 éq.) du dimercaptan.

Parallèlement, on prépare une solution de 0,05 mole (1 éq.) de 1,3-dichloroacétone dissoute dans environ 25 ml d'éther anhydre.

Dans un tricol, on additionne simultanément ces deux solutions sous agitation, à température ambiante et sous atmosphère d'azote sur une période de quatre heures environ.

A la fin de l'addition, le mélange réactionnel est versé dans un mélange eau-glace-éther contenant 10 ml d'une solution d'hydroxyde de sodium à 10%.

La phase aqueuse est extraite avec trois fois 40 ml d'éther. Le précipité blanc de polymère est décanté. Les phases organiques sont combinées, séchées sur sulfate de sodium et concentrées sous pression réduite.

## Va : 1,4-dithiacycloheptan 6-one

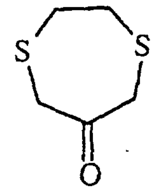

Rdt = 64%.
Eluant de purification : 5% acétate d'éthyle/95% d'éther de pétrole.

## VIIa : 1,5-dithiacyclooctan-7-one

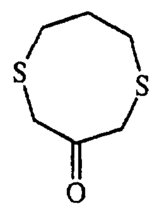

Rdt = 31%.
Eluant de purification : 5% acétate d'éthyle/95% d'éther de pétrole.

**II.2 Réduction par LiAlH4 dans le THF**

Le mode opératoire est le même que celui de l'étape I.4 ci-dessus.

## Vb : 1,4-dithiacycloheptan 6-ol

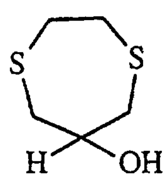

Rdt = 43%.
F = 65°C.
Eluant de purification : 15% acétate d'éthyle/85% éther de pétrole.

## VIIb : 1,5-dithiacyclooctan 7-ol

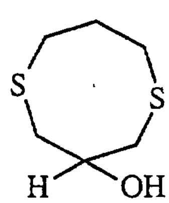

Rdt = 30%.
Eluant de purification : 10% acétate d'éthyle/90% éther de pétrole.

**II.3 Synthèse de mercaptans à partir d'alcools : utilisation du réactif de Lawesson**

Le mode opératoire est le même que celui de l'étape I.5 ci-dessus.

**VIa : 1,4-dithiacycloheptan 6-thiol**

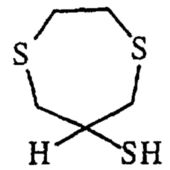

**II.4 Condensation du CMAO sur un alcool**

Le mode opératoire est le même que celui de l'étape I.6 ci-dessus.

**Vc**

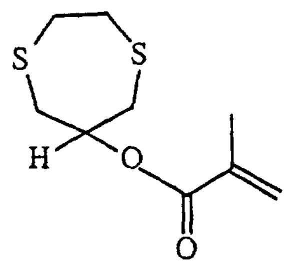

Rdt = 69%.
Eluant de purification : 5% acétate d'éthyle/95% éther de pétrole.

**VIIc**

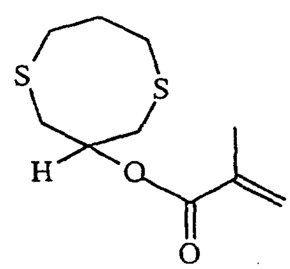

Rdt = 23%.
Eluant de purification : 2% acétate d'éthyle/98% éther de pétrole.

**II.5 Réaction du CMAO sur un mercaptan en présence d'une base tertiaire**

Dans un réacteur muni d'un thermomètre, d'une ampoule à brome et sous atmosphère d'azote, on introduit 55 mmoles de CMAO dilué dans 25 ml de solvant (acétonitrile, acétone ou toluène) auquel on ajoute 800 ppm d'EMHQ. On refroidit le mélange réactionnel à -10°C, et on ajoute goutte à goutte le mélange : mercaptan de l'étape II.3 (50

mmoles)/triéthylamine (55 mmoles) dilué dans 10 ml de solvant.

L'agitation est maintenue à la même température pendant cinq heures.

Après filtration du sel formé, le solvant est éliminé. Le résidu est repris au dichlorométhane, et lavé avec une solution d'hydroxyde de sodium 0,5 N; la phase organique est séchée sur sulfate de sodium et concentrée sous pression réduite.

Les thioesters méthacryliques ainsi obtenus sont purifiés par chromatographie sur gel de silice.

**VIb**

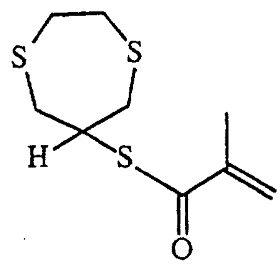

Rdt = 30%.

Eluant de purification : 1% acétate d'éthyle/99% éther de pétrole.

**III. Préparation de cycles soufrés contenant le motif S-S**

Les différentes étapes de cette synthèse sont :

- le traitement d'un dérivé dihalogéné par le disulfure de sodium ($Na_2S_2$) permet l'obtention du disulfure cyclique. Cette méthode utilise une réaction en catalyse par transfert de phase,
- puis la condensation du chlorure de méthacryloyle sur l'alcool précédemment obtenu.

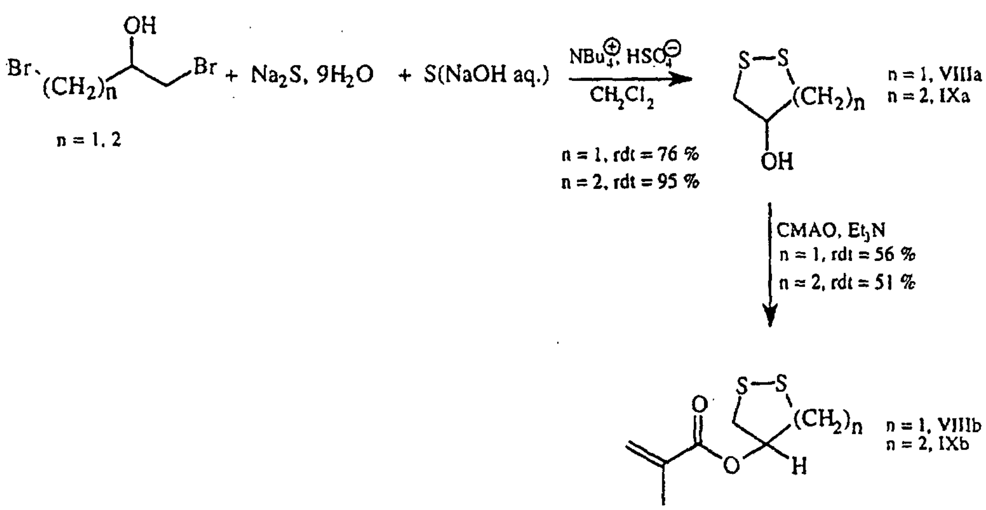


MODES OPERATOIRES

**III.1 Préparation des alcools VIIIa et IXa**

Dans un tricol muni d'un thermomètre et d'un réfrigérant, on dissout 1/4 de mole de $Na_2S$, $9H_2O$, dans 100 ml d'eau en portant cette solution à 40°C. On ajoute alors 3 pastilles de soude, puis du soufre, de manière à obtenir le système $Na_2S_2$ désiré (8 g, 1/4 mole pour n=2).

On laisse revenir à température ambiante, puis on ajoute 1/4 mole de dérivé dibromé dans 100 ml de dichlorométhane. Le tétrabutyl-ammonium hydrogénosulfate (catalyseur de transfert de phase) est alors additionné (5% en mole par rapport au dérivé halogéné).

Cette solution est portée à reflux pendant une heure sous agitation, puis on laisse revenir à température ambiante pendant une heure. La phase organique est reprise avec du dichlorométhane, puis lavée à l'eau, et enfin séchée sur sulfate de sodium. Le solvant est alors évaporé sous pression réduite. Le cycle ainsi obtenu est purifié par chromatographie sur gel de silice.

**VIIIa : 1,2-dithiacyclopentan 4-ol**

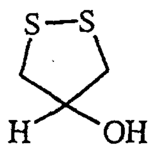

Rdt = 77%.
Eluant de purification : 20% acétate d'éthyle/80% éther de pétrole.

**IXa : 1,2-dithiacyclohexan 4-ol**

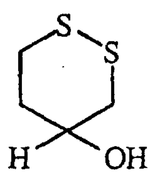

Rdt = 95%.
Eluant de purification : 20% acétate d'éthyle/80% éther de pétrole.

**III.3 Réaction d'addition du CMAO sur un alcool**

Le mode opératoire est le même que celui de l'étape I.6 ci-dessus.

**VIIIb**

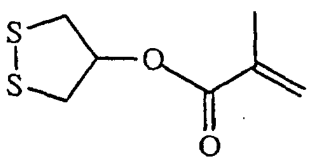

Rdt = 56%.
Eluant de purification : 2% acétate d'éthyle/98% éther de pétrole.

**IXb**

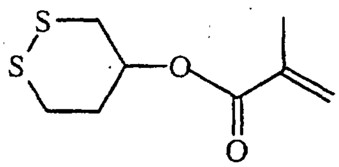

Rdt = 50%.
Eluant de purification : 2% acétate d'éthyle/98% éther de pétrole.

## IV. Synthèse d'un monomère méthacrylique dérivé du trithiane

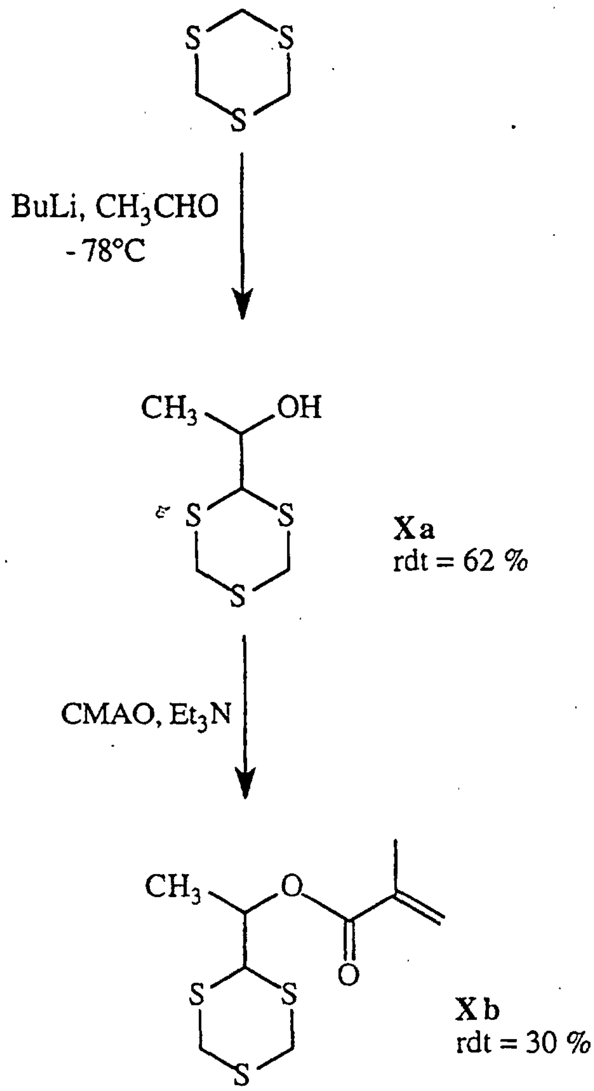


### MODES OPERATOIRES

### IV.1 Réaction d'addition d'un électrophile sur le trithiane

Dans un tricol, et sous atmosphère d'azote, on introduit 5 g (36 mmoles) de trithiane en solution dans 70 ml de tétrahydrofuranne anhydre. Le mélange réactionnel est refroidi à -30°C, température à laquelle on additionne lentement, 1,05 éq. de nBuLi (1,6M en solution dans l'hexane). Cette étape est exothermique et le mélange réactionnel prend une coloration jaune.

La température est maintenue entre -25 et -15°C pendant deux heures et trente minutes. A l'issue de cette période, le trithiane doit être entièrement dissout. Enfin, le mélange réactionnel est refroidi à -70°C, puis l'aldéhyde en solution dans le tétrahydrofuranne est additionné goutte à goutte à l'aide d'une seringue. Le mélange réactionnel est agité pendant une nuit à une température comprise entre 0 et 25°C.

L'agitation est maintenue encore pendant une heure à température ambiante et le mélange réactionnel est

versé dans un mélange $H_2O/CCl_4$. La phase aqueuse est extraite trois fois avec du tétrachlorure de carbone. On rassemble les phases organiques et le trithiane en suspension est filtré. La phase organique est lavée trois fois avec de l'eau pui séchée, filtrée et concentrée sous pression réduite.

**Xa**

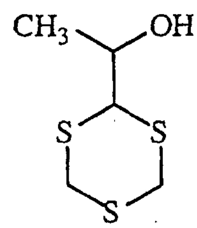

Rdt = 62%.

**IV.2 Condensation du CMAO sur l'alcool du trithiane**

Le mode opératoire est le même que celui de l'étape I.6 ci-dessus.

**Xb**

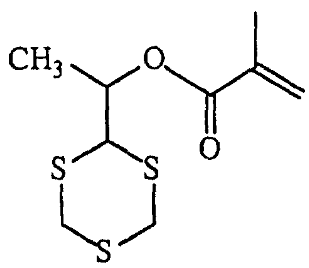

Rdt = 30%.
Eluant de purification : 2% acétate d'éthyle/98% éther de pétrole.

V. Synthèse de monomères méthacryliques dérivés du dithiane

## Premier schéma de synthèse

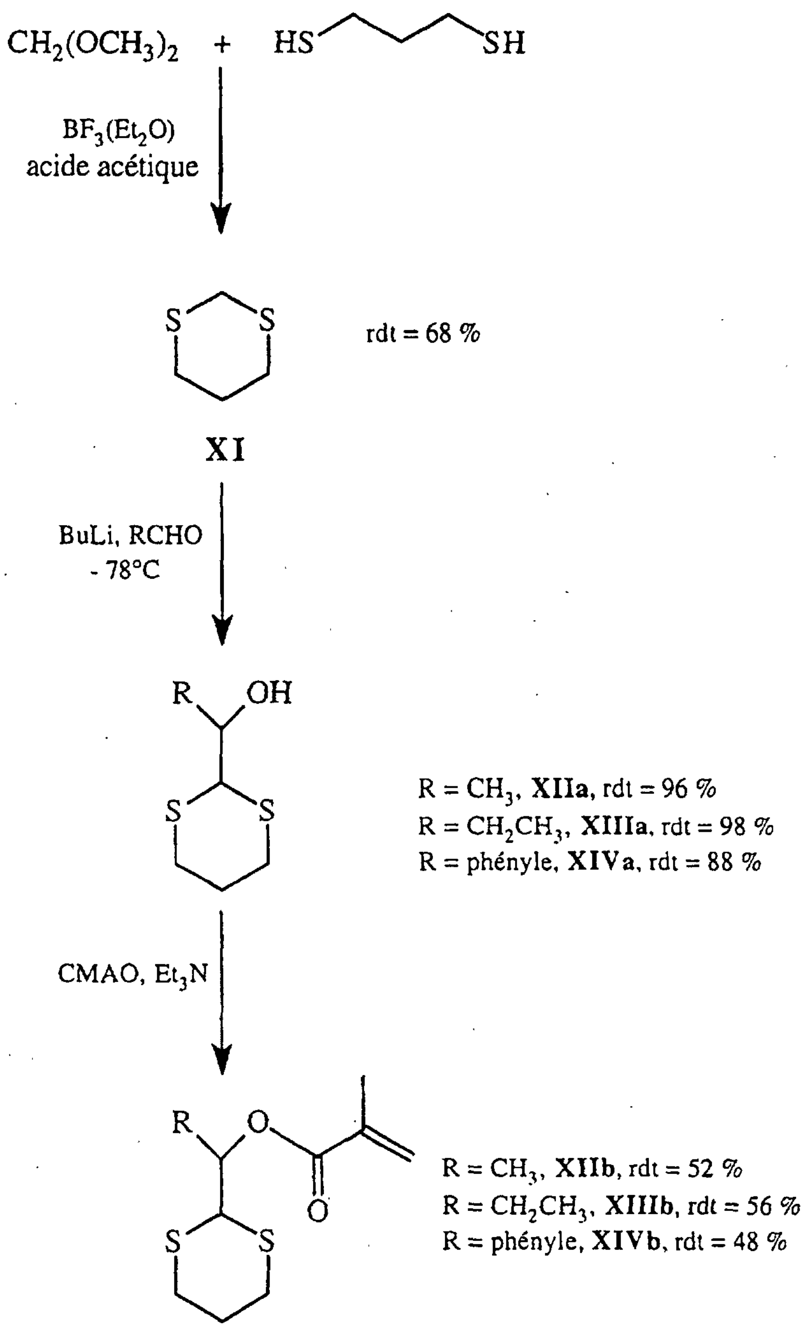

## Second schéma de synthèse

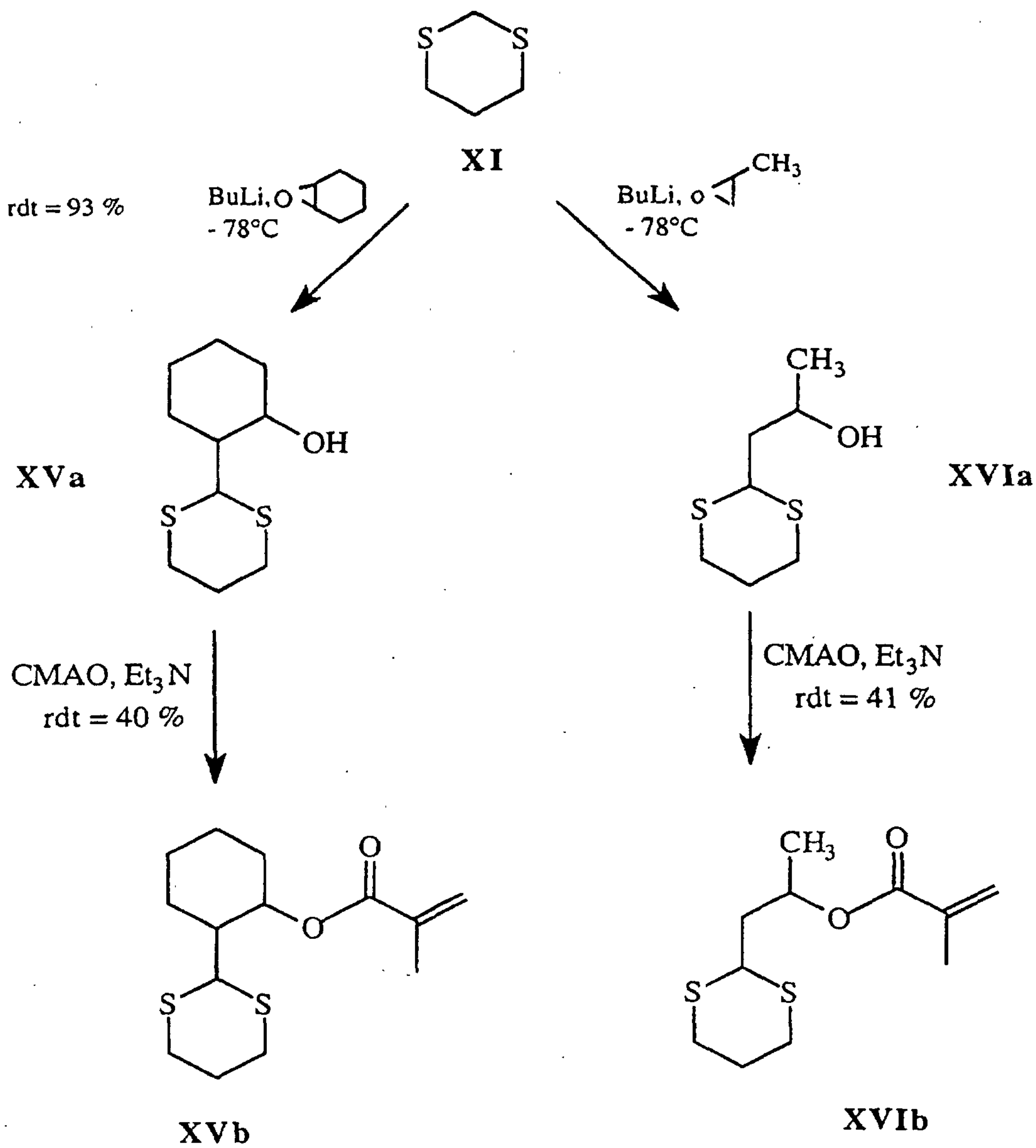


**MODES OPERATOIRES**

**V.1 Synthèse du dithiane**

Dans un tricol, muni d'un réfrigérant, d'une agitation magnétique et d'une ampoule à brome, on introduit 36 ml de $BF_3$ étherate, 72 ml d'acide acétique glacial dans 120 ml de chloroforme. Le mélange réactionnel est porté à reflux, puis on additionne goutte à goutte le mélange : 30 ml (0,3 mole) de propanedithiol, 29 ml de diméthoxy-méthane (0,33 mole) en solution dans 450 ml de chloroforme. L'addition s'effectue lentement sur une période de huit heures. On laisse remonter à température ambiante, puis le mélange réactionnel est lavé successivement avec quatre fois 80 ml d'eau, deux fois 120 ml d'une solution d'hydroxyde de potassium à 10% et à nouveau avec deux fois 80 ml d'eau. La phase organique est séchée sur sulfate de sodium, filtrée, puis le solvant est évaporé sous pression réduite.

Le résidu solide est repris avec 60 ml de méthanol, et chauffé au point d'ébullition du méthanol. On effectue une filtration à chaud, on laisse remonter à température ambiante et enfin, on garde cette solution à -20°C pendant une nuit. Les cristaux blancs de dithiane sont récupérés par filtration et séchés.

**XI : 1,3-dithiane**

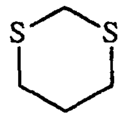

Rdt = 68%.
Point de fusion = 55°C.

**V.2 Réaction d'addition d'un électrophile sur le 1,3-dithiane**

Dans un tricol, et sous atmosphère d'azote, on introduit 5 g (41,6 mmoles) de dithiane en solution dans 80 ml de tétrahydrofuranne anhydre.
Le mélange réactionnel est refroidi à -40°C, température à laquelle on additionne goutte à goutte 27,3 ml (43,68 mmoles, 1,05 éq.) de nBuLi (1,6M en solution dans l'hexane). Le milieu réactionnel est ensuite agité pendant deux heures à une température comprise entre -20 et -40°C. A l'issue de cette période, on refroidit à -70°C pour additionner lentement l'électrophile (aldéhyde ou époxyde) en solution dans un minimum de tétrahydrofuranne.
Dans le cas d'un aldéhyde, la réaction est instantanée. Dans le cas d'un époxyde, la réaction est suivie par chromatographie sur couche mince.
Une fois la réaction terminée, le mélange réactionnel est hydrolysé lentement à froid avec de l'eau.
La phase aqueuse est extraite trois fois avec de l'éther.
Les phases organiques sont combinées, lavées trois fois avec de l'eau, puis avec une solution saturée en chlorure de sodium.
Les phases organiques sont séchées sur sulfate de sodium, puis le solvant est évaporé sous pression réduite.
Le produit ainsi obtenu est purifié par chromatographie sur gel de silice.

Electrophile : acétaldéhyde

**XIIa**

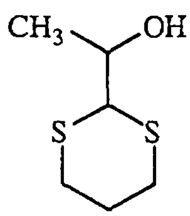

Rdt = 96%.
Eluant de purification : 10% acétate d'éthyle/90% éther de pétrole.

Electrophile : propionaldéhyde

**XIIIa**

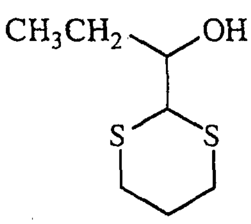

Rdt = 98%.

Eluant de purification : 10% acétate d'éthyle/90% éther de pétrole.

Electrophile : benzaldéhyde

**2-(hydroxyphénylméthyl) 1,3-dithiane**

**XIVa**

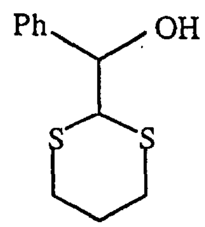

Rdt = 88%.
F = 73°C
Eluant de purification : 12% acétate d'éthyle/88% éther de pétrole.

Electrophile : oxyde de cyclohexène

**XVa**

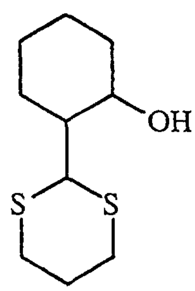

Rdt = 93%.
Eluant de purification : 10% acétate d'éthyle/90% éther de pétrole.

Electrophile : oxyde de propylène

**XVIa**

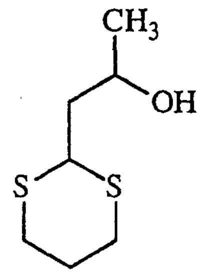

Rdt = 72%.

Eluant de purification : 11% acétate d'éthyle/89% éther de pétrole.

V.3 Condensation du CMAO sur l'alcool

Le mode opératoire est le même que celui de l'étape I.6 ci-dessus.

**XIIb**

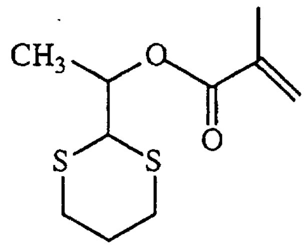

Rdt = 52%.
Eluant de purification : 2% acétate d'éthyle/98% éther de pétrole.

**XIIIb**

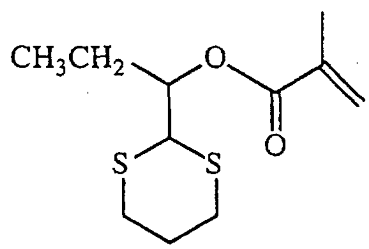

Rdt = 56%.
Eluant de purification : 3% acétate d'éthyle/97% éther de pétrole.

**2-(méthacrylate de phénylméthyl)-1,3-dithiane**

**XIVb**

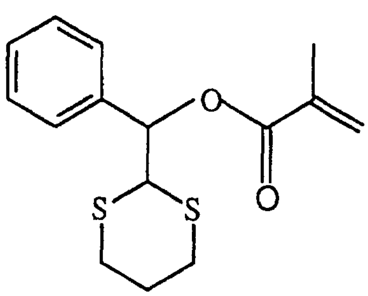

Rdt = 48%.
F = 97°C
Eluant de purification : 5% acétate d'éthyle/95% éther de pétrole.

**XVb**

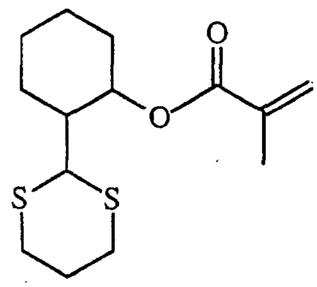

Rdt = 40%.
Eluant de purification : 2% acétate d'éthyle/98% éther de pétrole.

**XVIb**

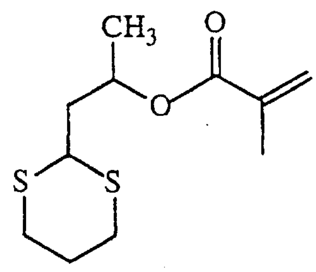

Rdt = 41 %.
Eluant de purification : 3% acétate d'éthyle/97% éther de pétrole.

VI. Synthèse de monomères méthacryliques dérivés d'alkyl dithiane

1ère voie de synthèse

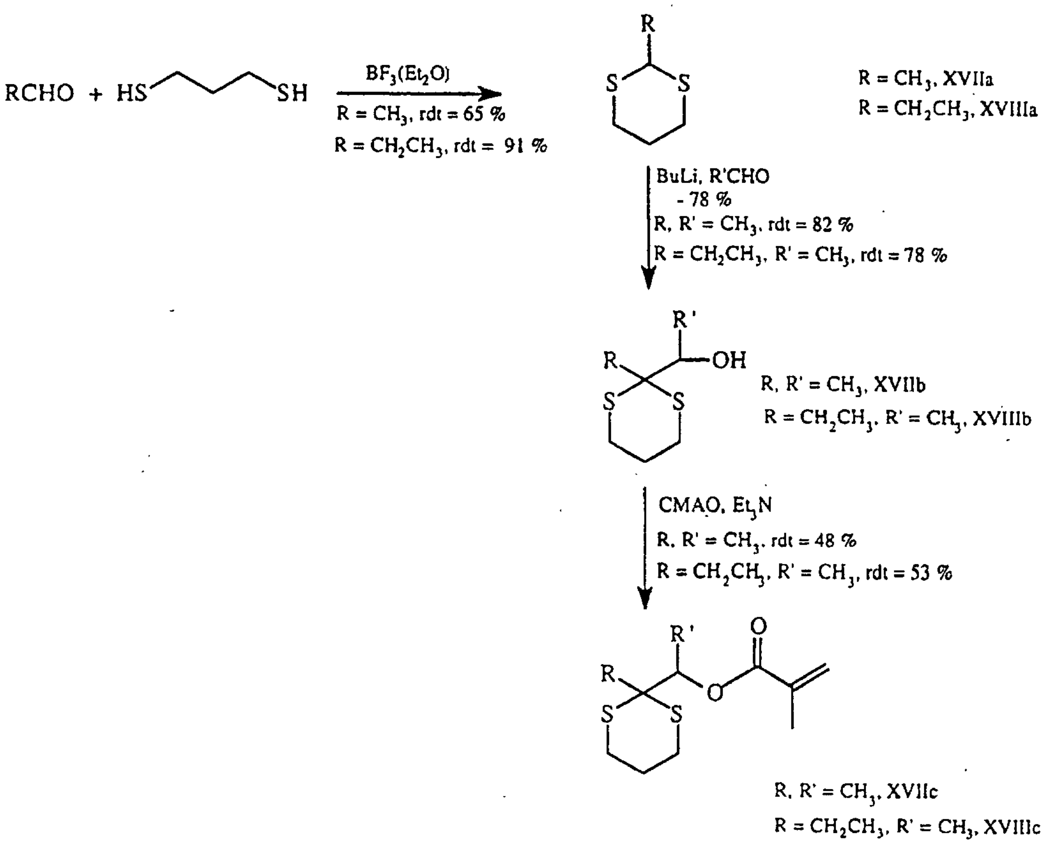

2ème voie de synthèse

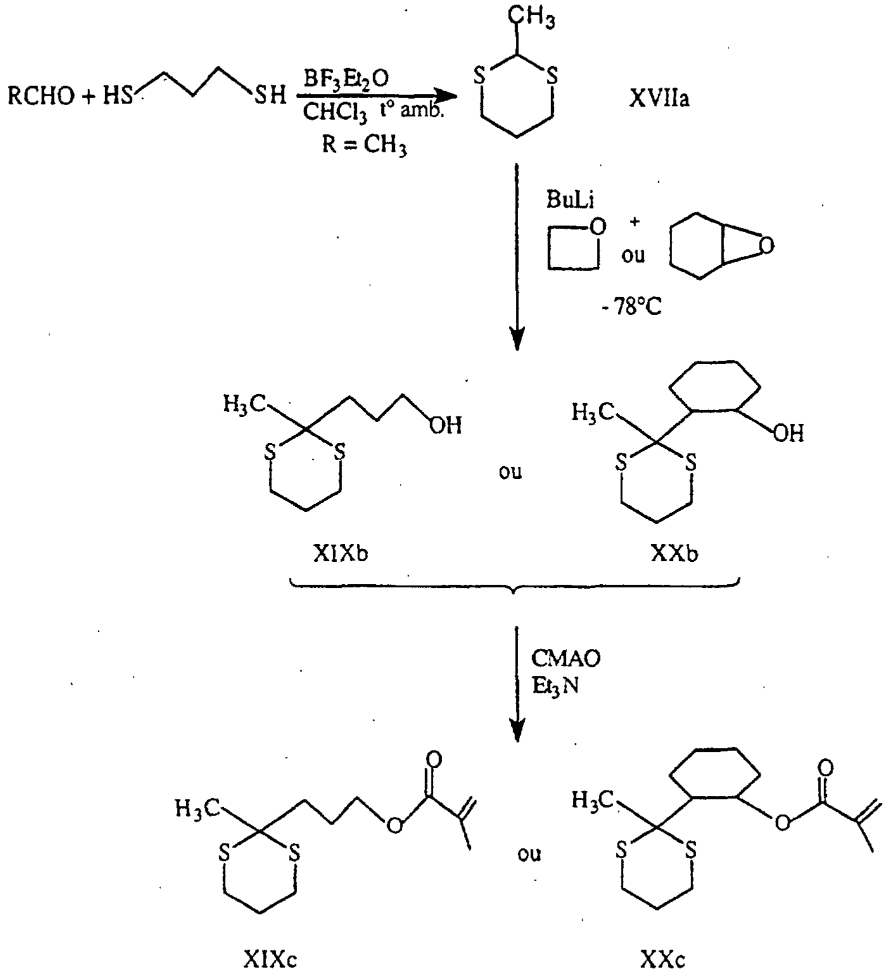


XVIIa

XIXb ou XXb

XIXc ou XXc

**VI.1 Préparation du 2-méthyl 1,3-dithiane et du 2-éthyl 1,3-dithiane**

Dans un tricol, on introduit 5 g (46,2 mmoles) de 1,3-propanedithiol, et 46,2 mmoles d'aldéhyde (acétaldéhyde ou propionaldéhyde) dans 60 ml de chloroforme. Cette solution est agitée pendant une heure à une température de -20°C. Ensuite, on additionne lentement 46,2 mmoles de $BF_3$ étherate, et on laisse remonter à température ambiante pendant quinze heures.

Le mélange réactionnel est lavé trois fois à l'eau, puis avec une solution d'hydroxyde de potassium à 10%. La phase organique est séchée sur sulfate de sodium, filtrée puis concentrée sous pression réduite.

## XVIIa : 2-méthyl 1,3-dithiane

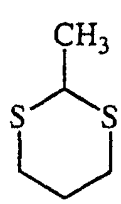

Rdt = 85%.

Eluant de purification : 3% acétate d'éthyle/97% éther de pétrole.

## XVIIIa : 2-éthyl 1,3-dithiane

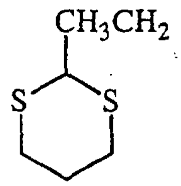

Rdt = 81%.
Eluant de purification : 2% acétate d'éthyle/98% éther de pétrole.

**VI.2 Addition d'un électrophile sur l'alkyl dithiane**

Le mode opératoire est le même que celui de l'étape V.2 ci-dessus.

Electrophile acétaldéhyde

**XVIIb**

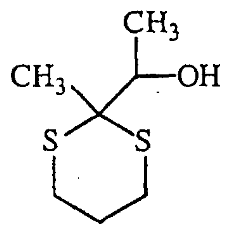

Rdt = 82%.
Eluant de purification : 9% acétate d'éthyle/81% éther de pétrole.

Electrophile acétaldéhyde

**XVIIIb**

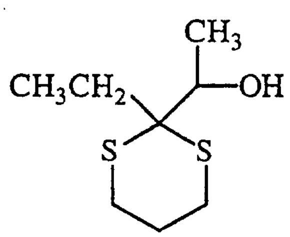

Rdt = 78%.
Eluant de purification : 9% acétate d'éthyle/91% éther de pétrole.

Electrophile : oxétane (oxyde de triméthylène) **2-méthyl, 2-(3'-hydroxypropyl-1')-1,3-dithiane**

**XIXb**

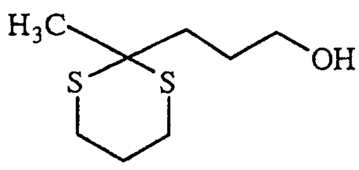

Rdt = 71%.

Electrophile : oxyde de cyclohexène **2-méthyl, 2-(2'-hydroxycyclohexyl-1') 1,3-dithiane**

**XXb**

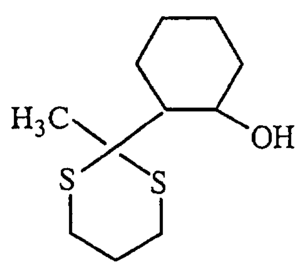

Rdt = 89%.

**VI.3 Addition du CMAO sur un alcool**

Le mode opératoire est le même que celui de l'étape I.6 ci-dessus.

**XVIIc**

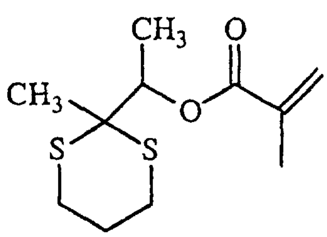

Rdt = 48%.
Eluant de purification : 1% acétate d'éthyle/99% éther de pétrole.

**XVIIIc**

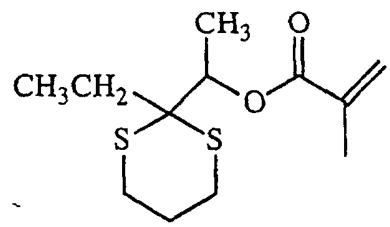

Rdt = 53%.
Eluant de purification : 4% acétate d'éthyle/96% éther de pétrole.

**2-méthyl, 2-(3'-méthacrylate de propyl-1')-1,3-dithiane**

**XIXc**

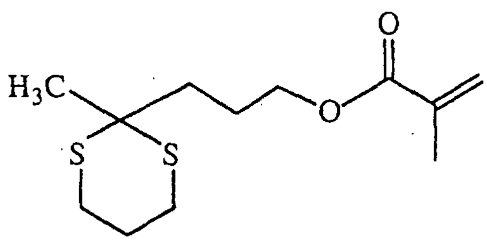

Rdt = 71%.
$n_D^{20}$ = 1,5268
$\nu_D$ = 39,9
Eluant de purification : 5% acétate d'éthyle/95% éther de pétrole.

2-méthyl, 2-(2'-méthacrylate de cyclohexyl-1')-1,3-dithiane

**XXc**

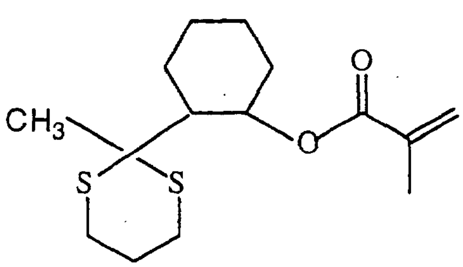

Rdt = 14%.
$n_D^{20}$ = 1,5425
$\nu_D$ = 41,7
Eluant de purification : 4% acétate d'éthyle/96% éther de pétrole.

Les propriétés optiques des monomères synthétisés ci-dessus ont été évaluées par mesure de leur indice de réfraction et de leur nombre d'ABBE. Les différents résultats sont indiqués dans le tableau I ci-dessous.

TABLEAU I

| Produits formule brute | Produits numéro | $n_D^{20}$ | $\nu_D$ |
|---|---|---|---|
| 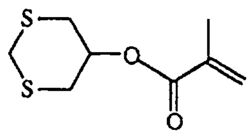 | **Id** | 1,5509 | 37,0 |
| 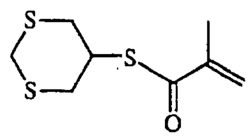 | **IIb** | 1,5960 | 31,6 |
| 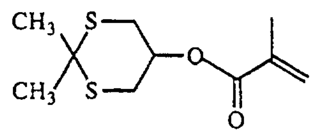 | **IIId** | 1,5215 | |
| 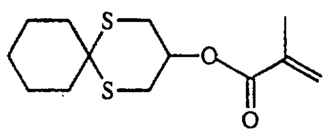 | **IVd** | 1,5462 | 40,1 |
| 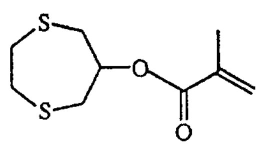 | **Vc** | 1,5447 | 39,5 |
| 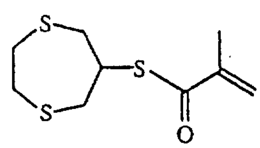 | **VIb** | 1,5923 | 33,9 |
| 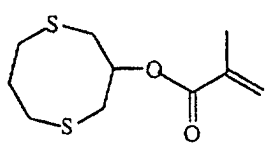 | **VIIc** | 1,5393 | 38,8 |
| 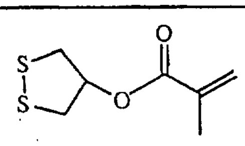 | **VIIIb** | 1,5163 | 38,0 |
| 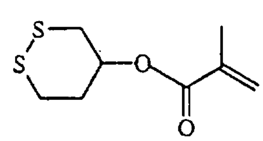 | **IXb** | 1,5419 | 36,0 |

## TABLEAU I (SUITE)

| | | | |
|---|---|---|---|
| 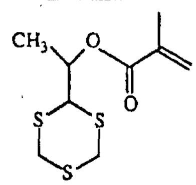 | **Xb** | 1,5690 | 36,9 |
| 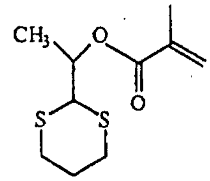 | **XIIb** | 1,5330 | 44,2 |
| 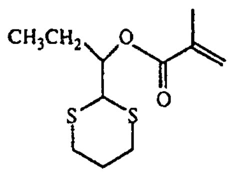 | **XIIIb** | 1,5202 | 39,5 |
| 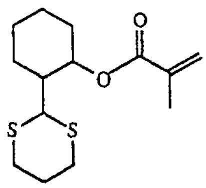 | **XVb** | 1,5288 | 40,2 |
| 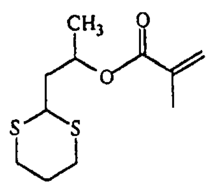 | **XVIb** | 1,5280 | 40,0 |
| 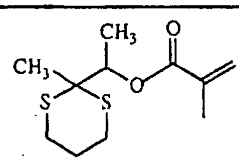 | **XVIIc** | 1,5300 | 39,2 |
| 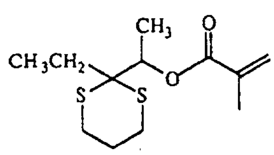 | **XVIIIc** | 1,5273 | 40,5 |
| 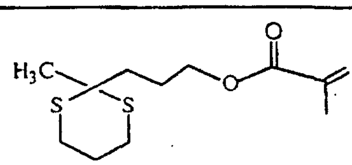 | **XIXc** | 1,5268 | 39,9 |
| 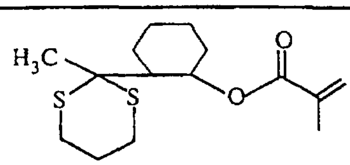 | **XXc** | 1,5425 | 41,7 |

**VII. Synthèse de dérivés du 1,3-dithioacétals à partir d'hydroxycétones.**

**Schéma récapitulatif**

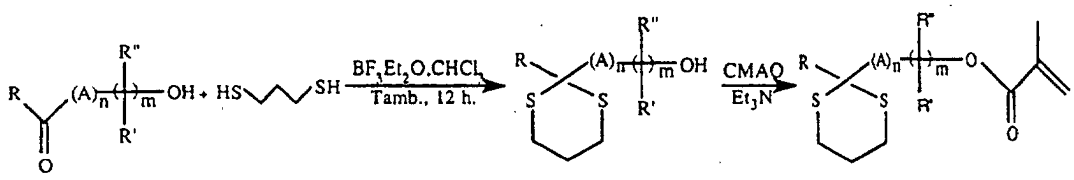


XXI XXIa

$A = CH_2$, Ph

n = 0, 1

R', R", R = H, $CH_3$, Ph

**VII - 1 Synthèse de 1,3-dithioacétals cycliques par réaction d'addition d'un dimercaptan sur une cétone.**

Dans un tricol, on introduit 46,2 mmoles de propanedithiol et 46,2 mmoles d'hydroxycétone dans 60 ml de chloroforme. Cette solution est agitée pendant 1 heure à une température de -20°C. Ensuite, on additionne lentement 46,2 mmoles de trifluorure de bore éthérate, et on laisse remonter à température ambiante pendant 12 heures.
Le mélange réactionnel est lavé trois fois à l'eau, puis avec une solution d'hydroxyde de potassium à 10%. La phase organique est séchée sur sulfate de sodium, filtrée, puis concentrée sous pression réduite. Dans le cas d'hydroxyphénylcétones, la phase organique ne subira aucun lavage basique. Les produits ainsi obtenus sont purifiés par chromatographie sur gel de silice.

**2-méthyl, 2-(2'-méthyl, 2'-hydroxypropyl-1')-1,3-dithiane**

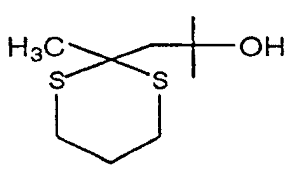

(XXII)

Rdt = 98%.

**2-méthyl, 2-(4'-hydroxyphényl-1')-1,3-dithiane**

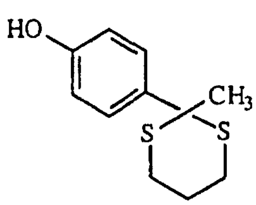

XXIII

Rdt = 98%.

**2-phényl-2-(4'-hydroxyphényl-1')-1,3-dithiane**

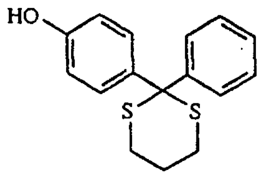

XXIV

Rdt = 95%

**VII - 2 Synthèse d'esters méthacryliques par condensation du chlorure de méthacryloyle sur un alcool**

Dans un tricol muni d'un réfrigérant, d'un thermomètre et d'une agitation magnétique, on introduit 50 mmoles (1 éq.) d'alcool, 50 mmoles (1 éq.) de triéthylamine et 800 ppm d'EMHQ dans 30 ml de chloroforme. Le mélange réactionnel est refroidi à 0°C; puis on additionne goutte à goutte 55 mmoles (1,1 éq.) de chlorure de méthacryloyle en solution dans 20 ml de chloroforme, tout en maintenant la température à 0°C. Après retour à température ambiante, on laisse agiter pendant 48 heures.

On acidifie le mélange réactionnel avec 50 ml d'une solution d'acide sulfurique 6N, et on extrait à l'éther. La phase organique est lavée successivement avec une solution d'hydrogénocarbonate de sodium à 10%, puis avec une solution saturée en chlorure de sodium. La phase éthérée est séchée sur sulfate de sodium, et le solvant est évaporé. Les composés méthacryliques ainsi obtenus sont purifiés par chromatographie sur gel de silice.

**2-méthyl, 2-(2'-méthyl, 2'-méthacrylate de propyl-1')-1,3-dithiane**

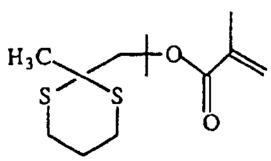

XXIIa

Rdt = 32%
$n_D^{20}$ = 1,5215
$\nu_D$ = 38,6
Eluant de purification : 1% acétate d'éthyle/99% éther de pétrole.

**2-méthyl, 2-(4'-méthacrylate de phényl-1')-1,3-dithiane**

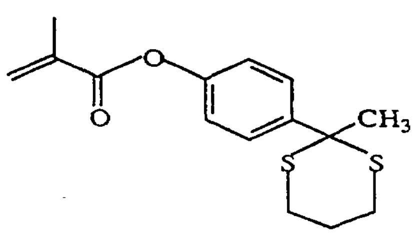

XXIIIa

Rdt = 50%

$n_D^{20}$ = 1,5742
$\nu_D$ = 30,8
Eluant de purification : 4% acétate d'éthyle/96% éther de pétrole.

**2-phényl, 2-(4'-méthacrylate de phényl-1')-1,3'-dithiane**

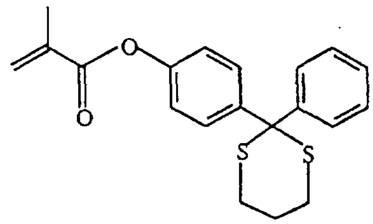

XXIVa

Rdt = 48%
F = 132°C
Eluant de purification : 2% acétate d'éthyle/98% éther de pétrole.

**<u>VIII - Synthèses du 2-méthyl 2-méthacrylate de méthyl 5-méthyl-(1,3-benzodithiol) et du 4-méthyl 4-hydroxyméthyl 3,5-dithiotricyclo [5,2,1,0 (2,6)] décane.</u>**

<u>Schéma de synthèse</u>

XXV XXVa

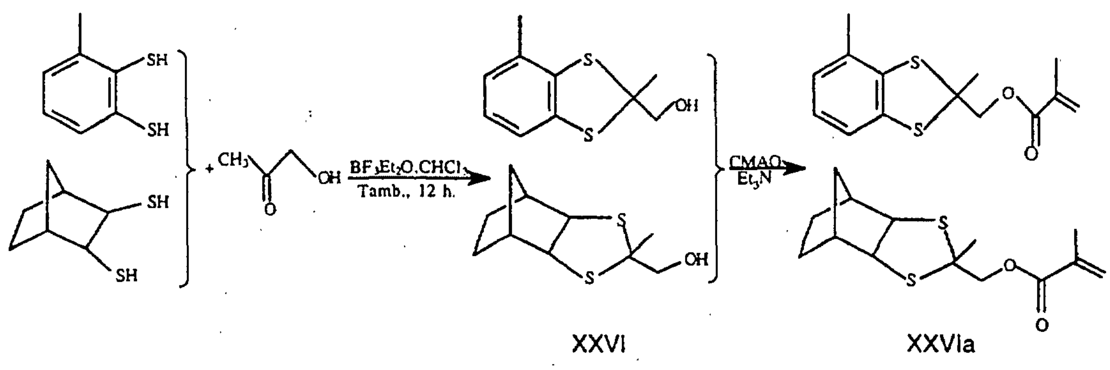


XXVI XXVIa

**<u>VIII - 1 Synthèse des alcools</u>**

Dans un tricol, on introduit 46,2 mmoles de dimercaptan et 46,2 mmoles d'hydroxyacétone dans 60 ml de chloroforme. Cette solution est agitée pendant 1 heure à une température de -20°C. Ensuite, on additionne lentement 46,2 mmoles de trifluorure de bore éthérate et on laisse remonter à température ambiante pendant 12 heures.

Le mélange réactionnel est lavé trois fois à l'eau, puis avec une solution d'hydroxyde de potassium à 10%. La phase organique est séchée sur sulfate de sodium, filtrée, puis concentrée sous pression réduite. Les produits ainsi obtenus sont purifiés par chromatographie sur gel de silice.

**2-méthyl 2-hydroxyméthyl 5-méthyl (1,3-benzodithiol)**

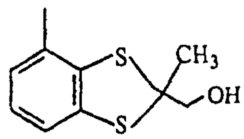

XXV

Rdt = quantitatif
Eluant de purification : 10% acétate d'éthyle/90% éther de pétrole.

**4-méthyl 4-hydroxyméthyl 3,5-dithiatricyclo [5,2,1,0$^{(2,6)}$] décane**

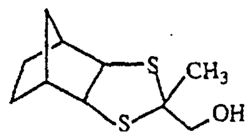

XXVI

Rdt = 67%

**<u>VIII - 2 Synthèse d'esters méthacryliques par condensation du chlorure de méthacryloyle sur un alcool</u>**

Dans un tricol muni d'un réfrigérant, d'un thermomètre et d'une agitation magnétique, on introduit 50 mmoles (1 éq.) d'alcool, 50 mmoles (1 éq.) de triéthylamine et 800 ppm d'EMHQ dans 30 ml de chloroforme. Le mélange réactionnel est refroidi à 0°C; puis on additionne goutte à goutte 55 mmoles (1 éq.) de chlorure de méthacryloyle en solution dans 20 ml de chloroforme, tout en maintenant la température à 0°C. Après retour à température ambiante, on laisse agiter pendant 48 heures.

On acidifie le mélange réactionnel avec 50 ml d'une solution d'acide sulfurique 6N et on extrait à l'éther. La phase organique est lavée successivement avec une solution d'hydrogénocarbonate de sodium à 10%, puis avec une solution saturée en chlorure de sodium. La phase éthérée est séchée sur sulfate de sodium, et le solvant est évaporé. Les composés méthacryliques ainsi obtenus sont purifiés par chromatographie sur gel de silice.

**2-méthyl 2-méthacrylate de méthyl 5-méthyl-(1,3-benzodithiol)**

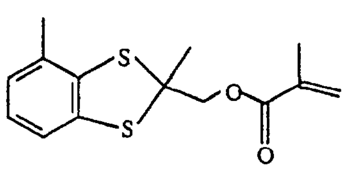

XXVa

Rdt = 38%
$n_D^{20}$ = 1,5706
$\nu_D$ = 27,5
Eluant de purification : 1% acétate d'éthyle/99% éther de pétrole.

**4-méthyl 4-méthacrylate de méthyl 3,5-dithiatricyclo [5,2,1,0$^{(2,6)}$] décane**

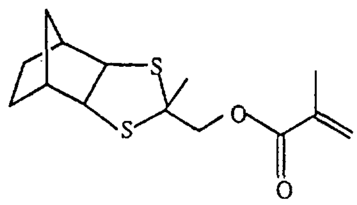

XXVIa

Rdt = 30%
$n_D^{20}$ = 1,5294
$\nu_D$ = 37,6
Eluant de purification : 1% acétate d'éthyle/99% éther de pétrole.

**IX - Synthèse du 2-(2',3'-diméthacrylate de propyl-1')-1,3-dithiane**

Schéma de synthèse

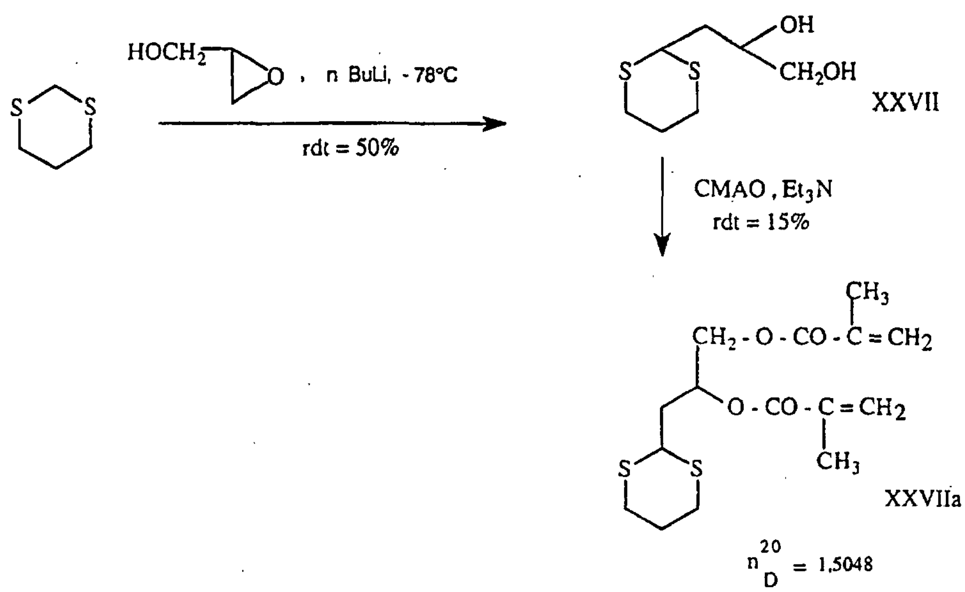


XXVII

XXVIIa

**Mode opératoire**

Le mode opératoire est analogue à celui décrit précédemment en **V.2** et **1.6.**
Les structures des monomères ont été confirmées par spectrographie RMN.
Les spectres RMN$^1$H ont été enregistrés à 250 Mhz sur un appareil BRUCKER AC 250. Les spectres RMN$^{13}$C découplés du proton ont été enregistrés à 62,88 Mhz sur l'appareil Brucker AC 250. La technique utilisée est l'Echo de Spin par Transformée de Fourier (SEFT).
Le tétraméthylsilane a été utilisé comme référence interne.
Les chromatographies sur couche mince ont été effectuées sur des plaques de silice (Kieselgel 60$F_{254}$) et révélées au moyen de permanganate de potassium ou d'iode.
Les spectres de masse ont été réalisés sur un appareil HEWLETT-PACKARD 5971A en impact électronique (tension d'ionisation : 70 eV). Le spectromètre est couplé à un chromatographe en phase gazeuse (colonne capillaire

de type WCOT Fused Silica, phase stationnaire : CP-Sil CB, longueur : 25 mètres, diamètre interne : 0,25 mm, épaisseur de film : 0,12 µm).

Les indices de réfraction ($n_D^{20}$) ont été mesurés à 20°C sur un réfractomètre d'ABBE (modèle ASTM-NFT 60194) pour la raie D du sodium (589,3 nm).

Les nombres d'ABBE ($\upsilon_D$) ont été calculés à partir des mesures d'indice de réfraction aux longueurs d'ondes suivantes : 480 nm (F' du Cadmium), 546,1 nm (E du Mercure), 589,3 nm (D du Sodium), 643,8 nm (C' du Cadmium).
$\upsilon_D$ se déduit par la formule :
$\upsilon_D = (n_D-1) / (n_{F'} - n_{C'})$.

Les solvants ont été distillés avant utilisation :

- l'éther anhydre séché sur $Na_2SO_4$, distillé sur sodium et conservée sur sodium.
- le tétrahydrofuranne anhydre distillé sur sodium en présence de benzophénone et conservé sur sodium.
- l'acétone anhydre séchée sur $CaCl_2$, distillée sur $KMnO_4$, séchée sur $K_2CO_3$ et conservée sur tamis moléculaire 4 Ångström.
- le chlorure de méthylène anhydre distillé sur $P_2O_5$ et conservé sur tamis moléculaire 4 Ångström.
- le chlorure de méthylène et l'éther distillés sur $P_2O_5$.
- le méthanol distillé sur magnésium.

La présente invention concerne également des composés nouveaux utiles comme intermédiaires pour la syntèse des monomères selon l'invention.

Plus particulièrement, ces nouveaux composés utiles comme intermédiaires de synthèse sont des composés thiol répondant à la formule :

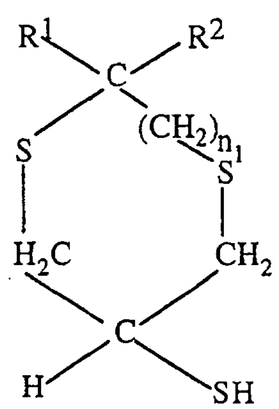

dans laquelle $R^1$, $R^2$ et $n_1$ sont définis comme précédemment. De préférence, $R^1$ et $R^2$ représentent tous deux un atome d'hydrogène.

Parmi ces composés nouveaux, on peut citer les composés de formules :

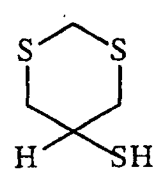

et

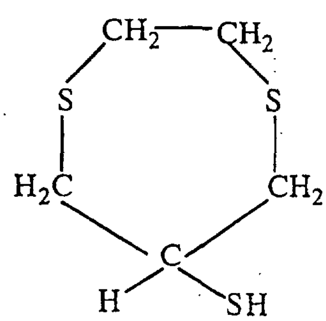

La présente invention concerne également des compositions polymérisables comportant au moins un monomère fonctionnel de type mono(thio)(méth)acrylate ou di(méth)acrylate, de préférence mono (thio)(méth)acrylate, porteur d'un hétérocycle constitué d'atomes d'hydrogène, carbone et soufre, ayant 5 à 8 chaînons et au moins deux

atomes de soufre intracycliques.

L'hétérocycle utile dans les compositions selon l'invention comporte de préférence 6 chaînons.

De préférence, également, l'hétérocycle du monomère utile dans les compositions polymérisables selon l'invention comporte deux atomes de soufre intracycliques en position 1-3 ou 1-4 de l'hétérocycle.

Dans une autre réalisation recommandée des compositions polymérisables selon l'invention, l'hétérocycle de monomère est un hétérocycle à 6 chaînons, comportant trois atomes de soufre intracycliques. Egalement, les monomères de type mono(thio)(méth)acrylate particulièrement recommandés des compositions polymérisables selon la présente invention sont des monomères thio(méth)acrylates. Enfin, ces monomères ont de préférence une masse molaire comprise entre 150 et 350, et mieux entre 200 et 300.

Les monomères de type mono(thio)(méth)acrylate particulièrement recommandés dans les compositions polymérisables de la présente invention, sont les monomères décrits précédemment et représentés par la formule (A), et tout spécialement ceux pour lesquels, dans la formule (A), X représente un atome de soufre.

Les compositions polymérisables selon l'invention peuvent comprendre uniquement un monomère fonctionnel suivant l'invention ou un mélange de ceux-ci, ou encore les compositions peuvent comporter un monomère ou un mélange de monomères selon l'invention tel que décrit précédemment, avec un ou plusieurs autres monomères usuels copolymérisables avec les monomères de l'invention pour la fabrication par polymérisation de polymères transparents ayant les propriétés optiques et/ou ophtalmiques appropriées.

On peut utiliser, dans les compositions polymérisables selon l'invention, tous comonomères convenables copolymérisables avec les monomères selon l'invention.

Parmi les comonomères utilisables avec les monomères de type (thio)(méth)acrylate des compositions polymérisables selon l'invention, on peut citer les monomères vinyliques, acryliques et méthacryliques mono- ou polyfonctionnels.

Parmi les comonomères vinyliques utiles dans les compositions de la présente invention, on peut citer les alcools vinyliques et les esters vinyliques tels que l'acétate de vinyle et le butyrate de vinyle.

Les comonomères acryliques et méthacryliques peuvent être des comonomères (méth)acrylates et d'alkyle mono- ou polyfonctionnels, mono(méth)acrylates polycycléniques ou aromatiques.

Parmi les (méth)acrylates d'alkyle, on peut citer le styrène, les $\alpha$-alkylstyrènes comme l'$\alpha$-méthylstyrène, le méthyl(méth)acrylate, l'éthyl(méth)acrylate, le butyl(méth)acrylate, l'isobutyl(méth)acrylate ou les dérivés difonctionnels tels que le butanediol diméthacrylate ou trifonctionnels tels que le triméthylolpropane triméthacrylate.

Parmi les comonomères mono(méth)acrylates polycycléniques, on peut citer le cyclohexyl(méth)acrylate, le méthylcyclohexyl (méth)acrylate, l'isobornyl(méth)acrylate et l'adamantyl(méth) acrylate.

On peut également citer comme comonomères les mono(méth) acrylates aromatiques tels que le phényl (méth)acrylate, le benzyl (méth)acrylate, le 1-naphtyl(méth)acrylate, le fluorophényl (méth)acrylate, le chlorophényl (méth)acrylate, le bromophényl (méth)acrylate, le tribromophényl(méth)acrylate, le méthoxyphényl (méth)acrylate, le cyanophényl(méth)acrylate, le biphényl (méth)acrylaté, le bromobenzyl(méth)acrylate, le tribromobenzyl (méth)acrylate, le bromobenzyléthoxy(méth)acrylate, le tribromobenzyléthoxy(méth)acrylate et le phénoxyéthyl(méth)acrylate.

Parmi les comonomères utilisables dans les compositions selon l'invention, on peut encore citer les allylcarbonates de polyols liquides aliphatiques ou aromatiques, linéaires ou ramifiés tels que les glycols aliphatiques de bis-allylcarbonate ou les alkylène bis(allylcarbonate). Parmi les polyols(allylcarbonate) qui peuvent être utilisés pour préparer les polymères transparents utilisables conformément à l'invention, on peut citer l'éthylèneglycol bis(allylcarbonate), le diéthylèneglycol bis(2-méthallylcarbonate), le diéthylèneglycol bis (allylcarbonate), l'éthylèneglycol bis(2-chloroallylcarbonate), le triéthylèneglycol bis(allylcarbonate), le 1,3-propanediol bis(allyl-carbonate), le propylèneglycol bis (2-éthylallylcarbonate), le 1,3-butanediol bis(allylcarbonate), le 1,4-butanediol bis(2-bromoallyl-carbonate), le dipropylèneglycol bis(allylcarbonate), le triméthylène-glycol bis(2-éthylallylcarbonate), le pentaméthylèneglycol bis(allyl-carbonate), l'isopropylène bisphénol bis(allylcarbonate).

Les comonomères utilisables dans les compositions selon l'invention comprennent également les esters de cellulose tels que l'acétate de cellulose, le propionate de cellulose et le butyrate de cellulose.

Des comonomères utilisables sont encore des monomères du type polyalkylèneglycoldi(méth)acrylate, ou des dérivés aromatiques di(méth)acrylate tels que le 2,2bis-4-méthacryloyloxypolyethoxyphénylpropane.

Les comonomères utiles dans la présente invention comprennent aussi des composés soufrés autres que ceux de formule (A). Il peut s'agir de mono ou poly(méth)acrylates porteurs d'un ou plusieurs atomes de soufre ou bien de monothio(méth)acrylates ou de polythio(méth)acrylates, par exemple tels que ceux décrits dans la demande de brevet EP-273710. Parmi les polythio(méth)acrylates, on peut citer le bis-2 méthacryloylthioéthylsulfure et le 4,4'bis-méthacryloylthiophénylsulfure.

Pour une description complémentaire des comonomères utilisables dans les compositions selon l'invention, on pourra se référer au brevet français n° 2 699 541.

La polymérisation des compositions polymérisables selon l'invention peut s'effectuer par tout procédé de polymérisation connu. Le procédé de polymérisation convenant particulièrement dans la présente invention est la

polymérisation photochimique. Un procédé de polymérisation recommandé est la polymérisation photochimique par le rayonnement ultraviolet et de préférence un rayonnement UV-A. Les conditions de polymérisation dépendent bien évidemment des monomères utilisés dans les compositions.

De tels procédés de polymérisation sont décrits, entre autres, dans le brevet FR-A-2 699 541.

Ainsi, les compositions polymérisables selon l'invention renferment également généralement des initiateurs de polymérisation, de préférence des photoinitiateurs, dans des proportions de 0,001 à 5% en poids par rapport au poids total de la composition, et de préférence, encore, de 0,01 à 1%.

Les photoinitiateurs utilisables dans les compositions polymérisables selon l'invention sont, notamment, l'oxyde de 2,4,6-triméthyl benzoyl diphénylphosphine, la 1-hydroxycyclohexyl phénylcétone, la 2,2-diméthoxy-1,2-diphényl éthane 1-one, et les alkylbenzoïne éthers.

Les photoinitiateurs recommandés sont la 1-hydroxycyclohexyl phényl cétone et l'oxyde de 2,4,6-triméthyl benzoyl diphénylphosphine.

Les compositions polymérisables selon l'invention peuvent également renfermer des additifs classiquement utilisés dans des compositions polymérisables destinées au moulage d'articles d'optique et ophtalmiques, en particulier des lentilles de contact, dans des proportions classiques, à savoir des inhibiteurs, des colorants, des absorbeurs UV, des parfums, des déodorants, des agents anti-oxydants et des agents anti-jaune.

La présente invention concerne également des compositions polymères transparentes obtenues par polymérisation et en particulier par photopolymérisation, des compositions polymérisables décrites précédemment.

La polymérisation s'effectue de manière connue en utilisant un mélange initial contenant les divers monomères de la composition polymérisable et les éventuels adjuvants, la réaction de polymérisation étant catalysable en utilisant des catalyseurs tels que du péroxyde de benzoyle, du péroxydicarbonate de cyclohexyle, du péroxydicarbonate de diisopropyle ou le 2,2'-azo-bis-butyronitrile.

De préférence, la polymérisation est une photopolymérisation et dans ce cas, les compositions polymérisables selon l'invention contiennent généralement des photoinitiateurs comme indiqué précédemment.

De préférence encore, cette photopolymérisation est une photopolymérisation par irradiation à la lumière ultra-violette.

L'invention concerne également des articles d'optique et ophtalmiques fabriqués à partir des compositions polymère transparentes selon l'invention, et en particulier des lentilles de contact.

Les compositions polymérisables selon l'invention peuvent conduire à l'obtention de polymères thermoplastiques. Dans ce cas, les compositions polymérisables conviennent particulièrement bien à l'obtention d'articles d'optique et ophtalmiques par moulage par injection (c'est-à-dire par compression dans un moule de la composition polymérisable portée à une température supérieure à sa température de transition vitreuse ou à point de fusion).

Toutefois, les compositions selon l'invention peuvent être utilisées pour l'obtention d'articles d'optique et ophtalmiques par tous procédés de moulage classiques.

En particulier, on peut obtenir des lentilles sous la forme définitive par coulée des compositions polymérisables entre deux moules présentant la géométrie de surface requise, puis polymérisation. On obtient alors une lentille dont les deux faces se trouvent dans leur état final. On peut également fabriquer des lentilles semi-finies comportant après moulage une seule face à sa géométrie finale, la deuxième face pouvant être alors surfacée à la demande.

## EXEMPLE DE REALISATION D'UN POLYMERE TRANSPARENT SELON LA PRESENTE INVENTION.

Le monomère est préparé à l'abri de la lumière ultra-violette.

Un initiateur est additionné au monomère à raison de 0,1% en poids. Cette solution est ensuite injectée dans le moule de polymérisation.

Puis la polymérisation est effectuée par irradiation ultra-violette.

Le suivi cinétique de polymérisation est réalisé grâce à un spectromètre proche infrarouge qui permet d'observer la disparition de la bande C=C de l'acrylique à 6200 $cm^{-1}$ pour les esters méthacryliques et environ 6140 $cm^{-1}$ pour les thioesters méthacryliques.

L'homopolymérisation de l'ester méthacrylique du 1,4-dithiacycloheptane a pu être réalisée dans de bonnes conditions et les caractéristiques du monomère et du polymère sont indiquées ci-dessous.

**MONOMERE** : ester méthacrylique du 1,4-dithiacycloheptane.

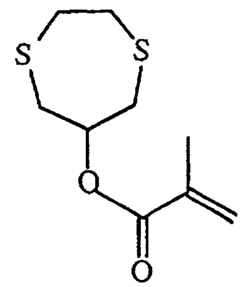

| | |
|---|---|
| **Propriétés** | $n_D^{20}$ = 1,5447<br>$n^b$ d'ABBE = 39,5<br>couleur transparente liquide stabilisée au bis-ditertiobutyl hydroxy toluène |

Homopolymérisation en présence de 0,1% d'initiateur. Suivi cinétique par proche infra-rouge.

| POLYMERE | |
|---|---|
| **Propriétés** | $n_D^{20}$ = 1,5892<br>$n^b$ d'ABBE = 43,5<br>couleur jaune et cassant<br>d = 1,24 |

**Revendications**

**1.** Composé monomère, **caractérisé en ce qu'**il répond à la formule :

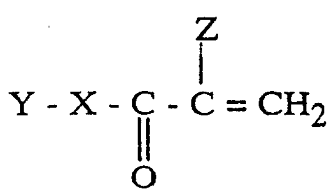

(A)

dans laquelle Z représente H ou $CH_3$ et X représente O ou S, et

- lorsque X représente S, Y est un radical de formule :

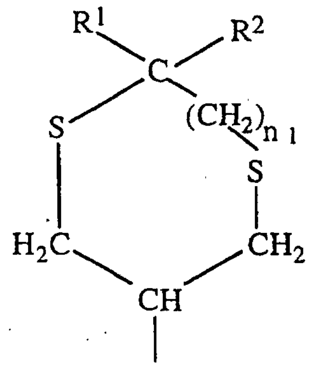 (a)

où $R^1$ et $R^2$ sont choisis parmi H, les radicaux alkyle, de préférence les radicaux alkyle en $C_1C_4$, et mieux le radical $CH_3$, ou encore $R^1$ et $R^2$ forment conjointement un radical

$$-(CH_2)_5-,$$

et $n_1$ est un entier de 0 à 2 inclus, et

- lorsque X représente O, Y est le radical (a) défini ci-dessus ou un radical choisi parmi les radicaux de formules :

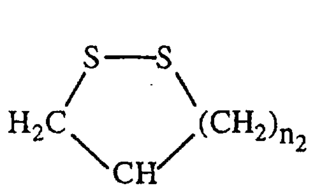 (b) , 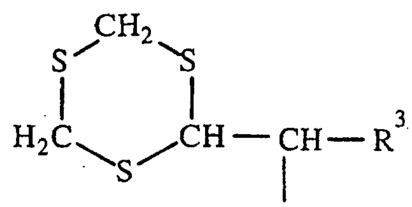 (c)

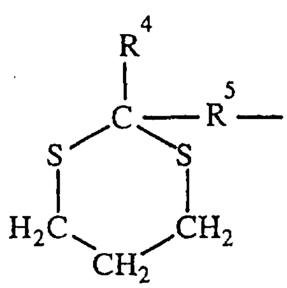 (d) et 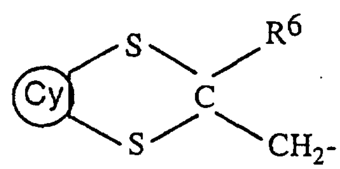 (e)

dans lesquelles $n_2$ est un entier égal à 1 ou 2, $R^3$ représente H ou un groupe alkyle, de préférence un groupe alkyle en $C_1$-$C_4$, et mieux un groupe méthyle, $R^4$ représente H, un groupe alkyle, un groupe aryle; le groupe alkyle étant de préférence un groupe alkyle en $C_1$-$C_4$, et mieux un groupe méthyle ou éthyle et le groupe aryle étant de préférence un groupe aryle en $C_6$-$C_{12}$, et mieux un groupe phényle, et $R^5$ est un radical divalent choisi parmi les groupements de formules suivantes :

(I) 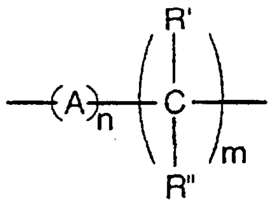

dans laquelle :

A désigne un groupe aryle, préférentiellement en $C_6$-$C_{12}$, et mieux un groupe phényle ou un groupe alkyle préférentiellement en $C_1$-$C_6$,
R', R" désignent indépendamment l'un de l'autre H, un groupe alkyle, préférentiellement un groupe alkyle en $C_1$-$C_6$, aryle, préférentiellement phényle, ou l'un de R', R" peut être un groupe

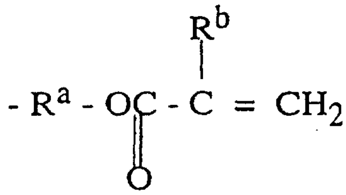

où $R^a$ est un groupe alkylène, de préférence en $C_1$-$C_6$, en particulier un groupe $-CH_2-$, et $R^b$ est H ou $CH_3$, n prend les valeurs O ou 1 et O ≤ m (entier ≤ 4), et

(II) 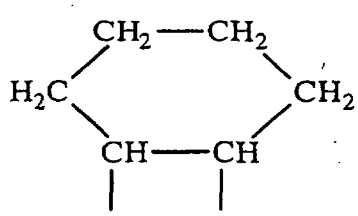 ,

et
$R_6$ désigne H ou un groupe méthyle, et
Cy désigne un noyau aryle substitué ou non, de préférence un noyau phényle, tolyle ou norbornyle.

2. Composé monomère monofonctionnel de type mono(thio) (méth)acrylate, selon la revendication 1, **caractérisé en ce que** $R^5$ est un radical divalent choisi parmi :

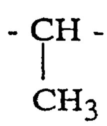 , 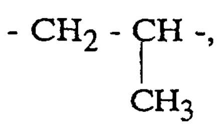 , 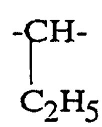

et

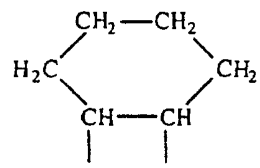

et Cy est un groupe phényle, tolyle ou norbornyle.

3. Composé monomère selon la revendication 1, **caractérisé en ce qu'**il est choisi parmi les composés de formules :

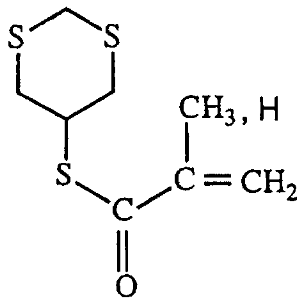
,

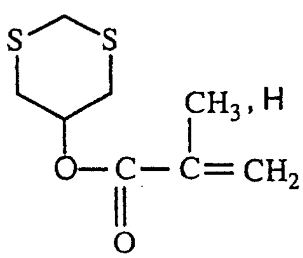
,
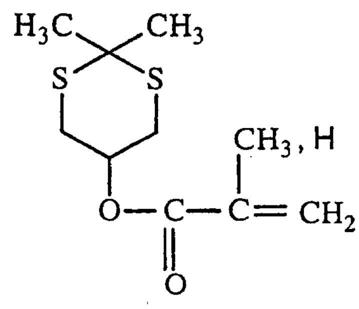
,

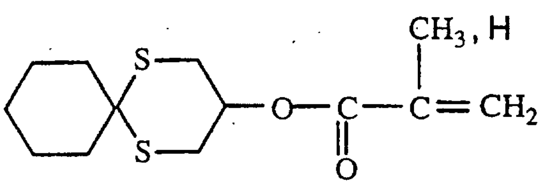
,
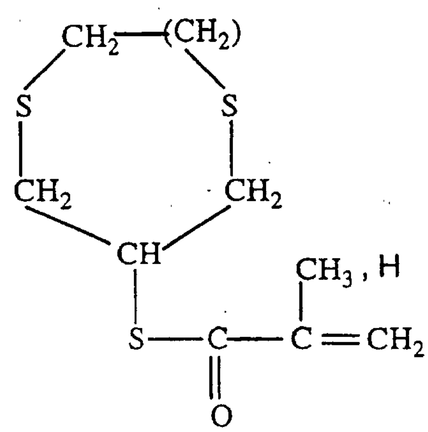
,

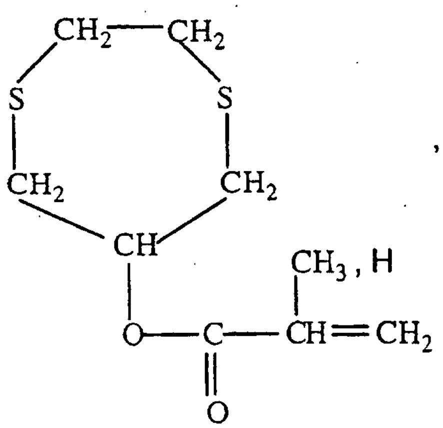

,

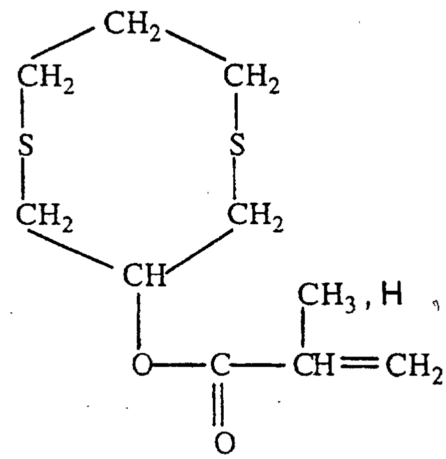

,

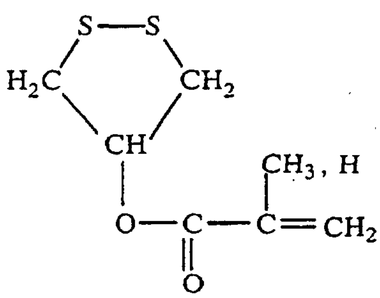

,

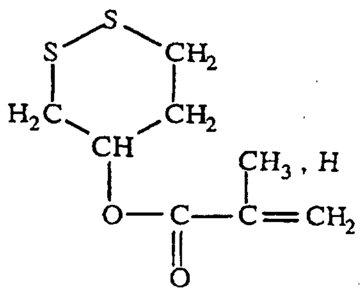

,

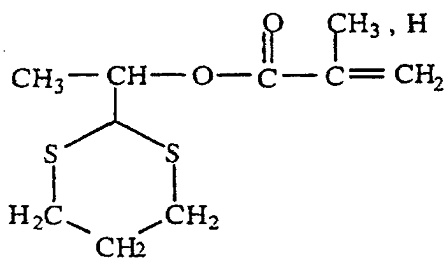

,

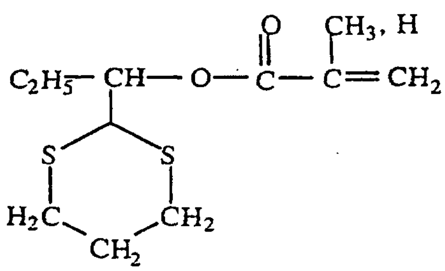

,

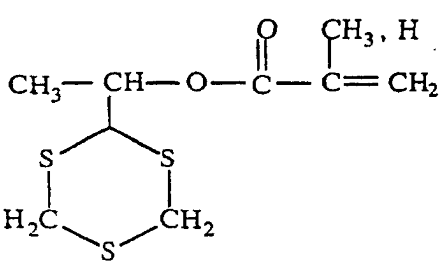

,

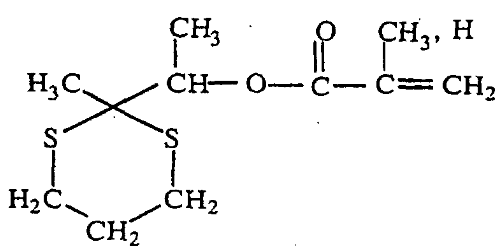

,

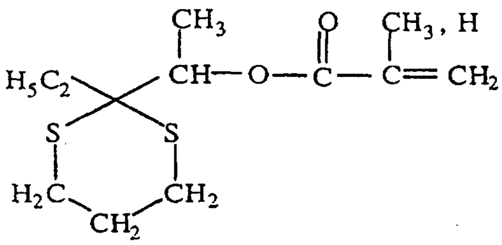

,

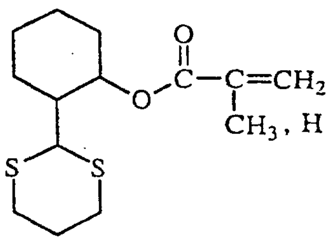

,

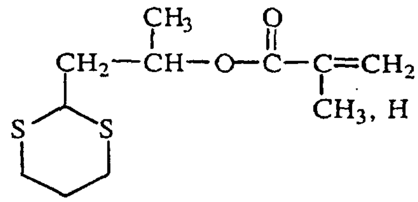

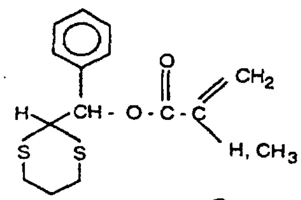

,

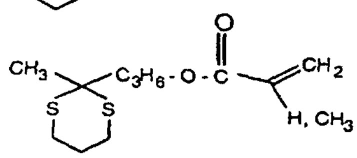

,

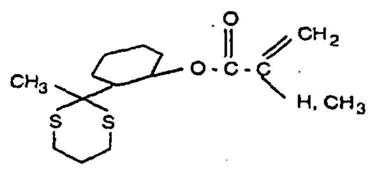

,

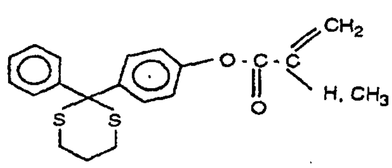

,

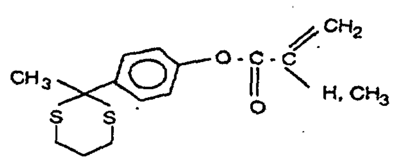

,

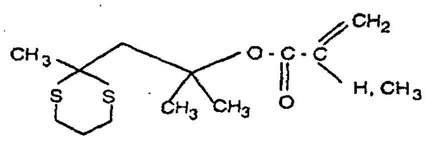

,

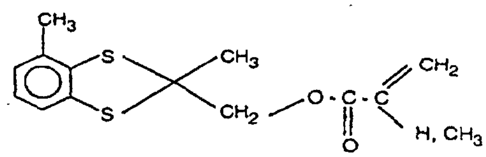

,

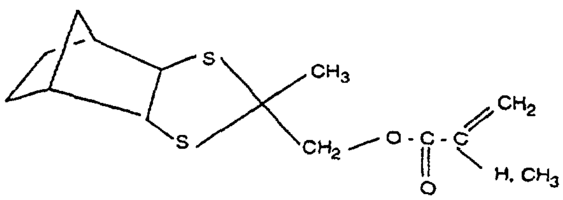

, et

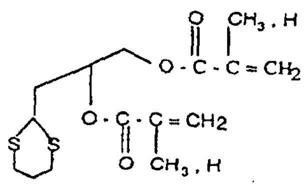

4. Composés utiles comme intermédiaires pour la synthèse des composés monomères selon la revendication 1, **caractérisés en ce qu'**ils répondent à la formule :

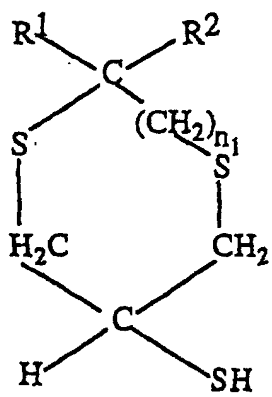

où $R^1$, $R^2$ et $n_1$ sont définis comme dans la revendication 1.

5. Composés selon la revendication 4, **caractérisés en ce qu'**ils sont choisis parmi les composés de formules :

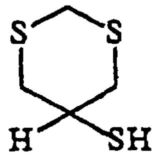 et 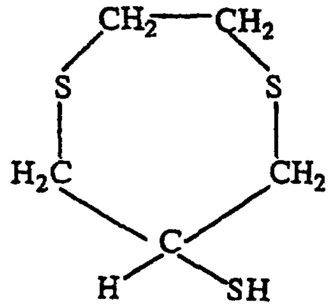

**6.** Composition polymérisable, **caractérisée en ce qu'**elle comprend au moins un monomère de type mono(thio)(méth)acrylate ou di(méth)acrylate porteur d'un hétérocycle constitué d'atomes de C, H et S ayant 5 à 8 chaînons et comportant 2 ou 3 atomes de soufre intracycliques, l'hétérocycle étant éventuellement condensé avec un cycle aromatique ou polycyclanique en $C_5$-$C_8$, substitué ou non, de préférence un cycle en $C_6$-$C_7$.

**7.** Composition polymérisable selon la revendication 6, **caractérisée en ce que** l'hétérocycle a 6 chaînons.

**8.** Composition polymérisable selon la revendication 6 ou 7, **caractérisée en ce que** le monomère a une masse molaire comprise entre 150 et 400, de préférence entre 150 et 350, mieux entre 200 et 300.

**9.** Composition polymérisable selon l'une quelconque des revendications 6 à 8, **caractérisée en ce que** l'hétérocycle comporte deux atomes de soufre intracycliques en position 1-3 ou 1-4 de l'hétérocycle.

**10.** Composition polymérisable selon l'une quelconque des revendications 6 à 8, **caractérisée en ce que** l'hétérocycle comporte trois atomes de soufre intracycliques.

**11.** Composition polymérisable selon l'une quelconque des revendications 6 à 9, **caractérisée en ce que** le monomère est de type thio(méth)acrylate.

**12.** Composition polymérisable, **caractérisée en ce qu'**elle comprend au moins un monomère selon la revendication 1, 2 ou 3.

**13.** Composition polymérisable selon l'une quelconque des revendications 6 à 12, **caractérisée en ce qu'**elle comprend en outre un ou plusieurs comonomères différents des monomères de formule (A).

**14.** Composition polymérisable selon la revendication 13, **caractérisée en ce que** le ou les comonomères sont choisis parmi les monomères vinyliques, acryliques, méthacryliques et les allylcarbonates de polyols liquides aliphatiques ou aromatiques, linéaires ou ramifiés.

**15.** Composition polymérisable selon l'une quelconque des revendications 6 à 14, **caractérisée en ce qu'**elle comprend en outre un initiateur de polymérisation.

**16.** Composition polymérisable selon la revendication 15, **caractérisée en ce que** l'initiateur de polymérisation est un initiateur de photopolymérisation.

**17.** Composition polymérisable selon la revendication 16, **caractérisée en ce que** le photoinitiateur est choisi parmi l'oxyde de 2,4,6-triméthyl benzoyl diphényl phosphine, la 1-hydroxycyclohexyl phényl cétone, la 2,2-diméthoxy-1,2-diphényl éthane-1-one, les alkyl benzoïne éthers et leurs mélanges.

**18.** Composition polymère transparente résultant de la polymérisation de l'une quelconque des compositions polymérisables, selon les revendications 6 à 17.

**19.** Composition polymère transparente résultant de la photopolymérisation de la composition polymérisable, selon la revendication 16 ou 17.

**20.** Article d'optique ou ophtalmique obtenu par moulage et polymérisation d'une composition, selon l'une quelconque

des revendications 6 à 17.

**21.** Article ophtalmique selon la revendication 20, **caractérisé en ce que** l'article est une lentille ophtalmique.

## Patentansprüche

**1.** Monomerverbindung, **dadurch gekennzeichnet, dass** sie der Formel

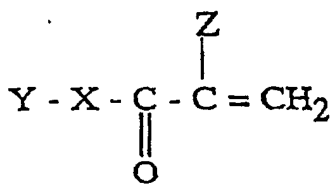

(A)

entspricht, wobei Z H oder $CH_3$ darstellt und X O oder S darstellt und folgendes gilt:

- wenn X S darstellt, ist Y ein Rest mit der Formel

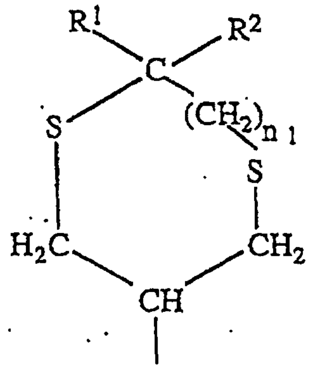

(a)

wobei $R^1$ und $R^2$ aus H, Alkylresten, vorzugsweise $C_1$-$C_4$-Alkylresten und besonders bevorzugt dem $CH_3$-Rest ausgewählt sind oder $R^1$ und $R^2$ zusammen einen Rest $-(CH_2)_5-$ bilden und $n_1$ eine ganze Zahl von 0 bis einschließlich 2 ist; und

- wenn X O darstellt, ist Y der oben definierte Rest (a) oder ein Rest, der aus Resten der folgenden Formeln ausgewählt ist:

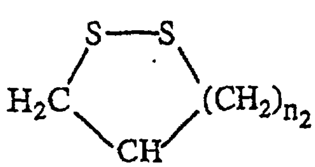

(b) ,

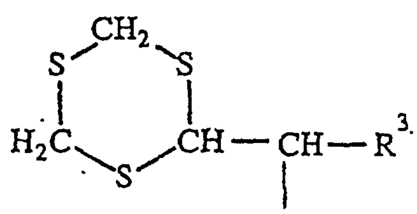

(c)

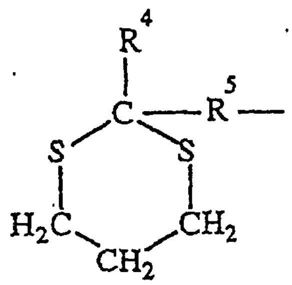 (d) und 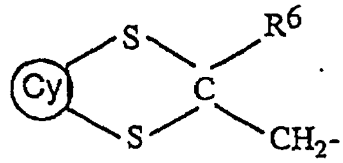 (e)

wobei $n_2$ eine ganze Zahl von 1 oder 2 ist, $R^3$ H oder eine Alkylgruppe, vorzugsweise eine $C_1$-$C_4$-Alkylgruppe und besonders bevorzugt eine Methylgruppe darstellt, $R^4$ H, eine Alkylgruppe oder eine Arylgruppe darstellt, wobei die Alkylgruppe vorzugsweise eine $C_1$-$C_4$-Alkylgruppe und besonders bevorzugt eine Methyl- oder Ethylgruppe ist und die Arylgruppe vorzugsweise eine $C_6$-$C_{12}$-Arylgruppe und besonders bevorzugt eine Phenylgruppe ist, und $R^5$ ein zweiwertiger Rest ist, der aus den Gruppen mit den folgenden Formeln ausgewählt ist:

(I) 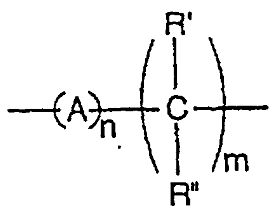

wobei:

A eine Arylgruppe, vorzugsweise eine $C_6$-$C_{12}$-Arylgruppe und besonders bevorzugt eine Phenylgruppe oder eine Alkylgruppe, vorzugsweise eine

$C_1$-$C_6$-Alkylgruppe, bedeutet;

R' und R" unabhängig voneinander H, eine Alkylgruppe, vorzugsweise eine $C_1$-$C_6$-Alkylgruppe, Aryl, vorzugsweise Phenyl, bedeuten oder eine der Gruppen R' und R" eine Gruppe

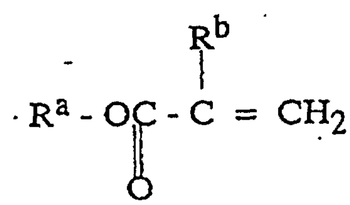

sein kann, wobei $R^a$ eine Alkylengruppe, vorzugsweise eine $C_1$-$C_6$-Alkylengruppe, insbesondere eine $-CH_2$-Gruppe, ist und Rb H oder $CH_3$ ist, n die Werte 0 oder 1 annimmt und $0 \leq m$ ist (ganze Zahl < 4); sowie

(II) 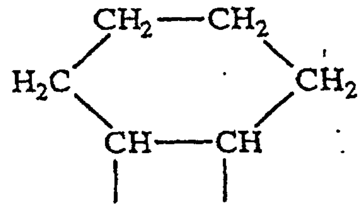

;

und

$R_6$ H oder eine Methylgruppe ist und

Cy einen gegebenenfalls substituierten Arylkern, vorzugsweise einen Phenyl-, Tolyl- oder Norbornylkern, bezeichnet.

**2.** Monofunktionelle Monomerverbindung des Typs Mono(thio)(meth)acrylat gemäß Anspruch 1, **dadurch gekennzeichnet, dass** $R^5$ ein zweiwertiger Rest ist, der aus

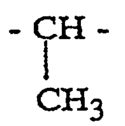 , 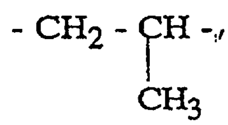 , 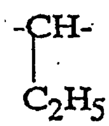 und 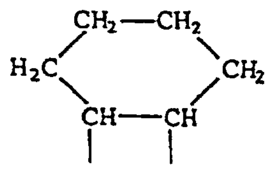

ausgewählt ist und Cy eine Phenyl-, Tolyl- oder Norbornylgruppe ist.

**3.** Monomere Verbindung gemäß Anspruch 1, **dadurch gekennzeichnet, dass** sie aus den Verbindungen mit den folgenden Formeln ausgewählt ist:

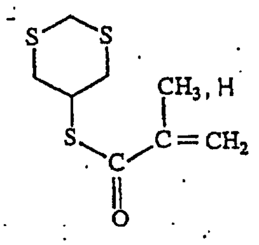 ,

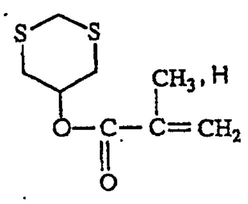 , 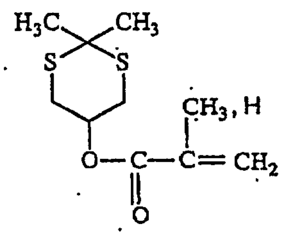 ,

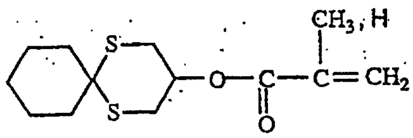 , 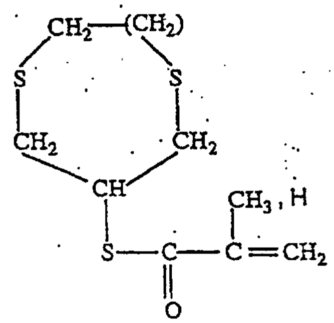 ,

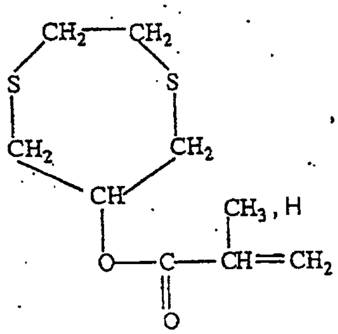 , 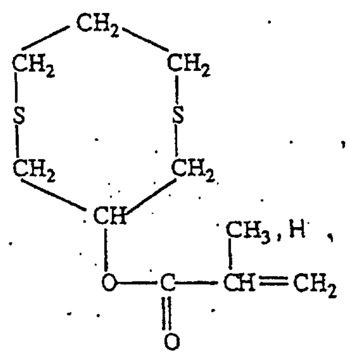 ,

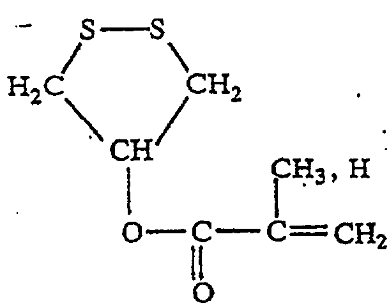 , 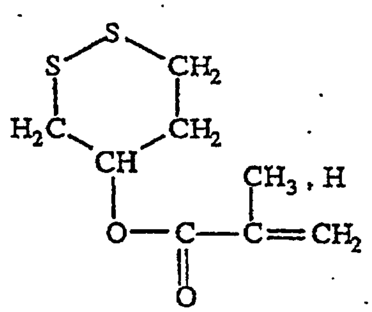 ,

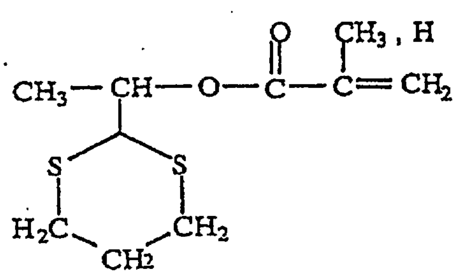 , 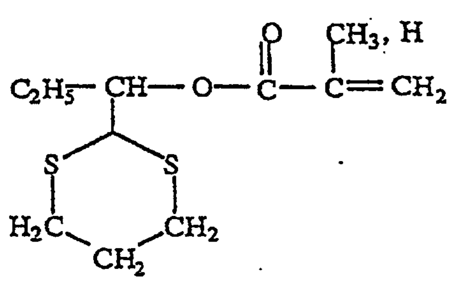 ,

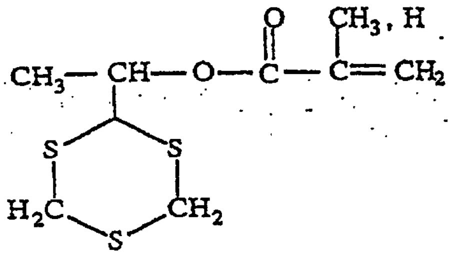
,
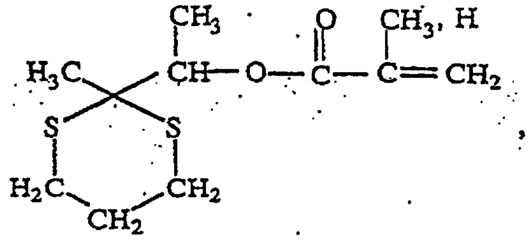
,

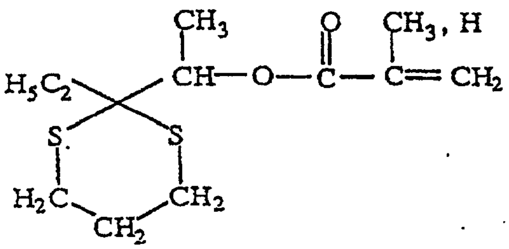
,
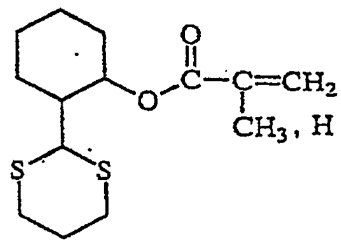

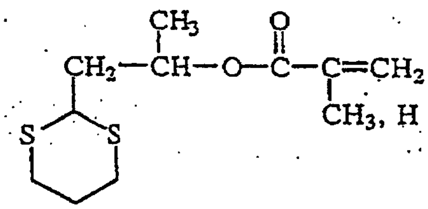

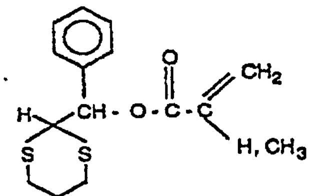

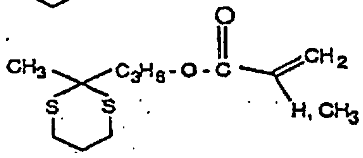

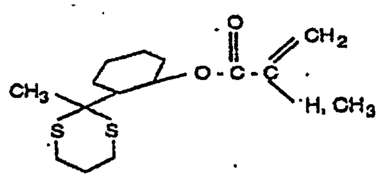

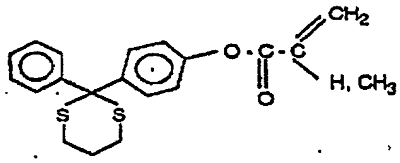

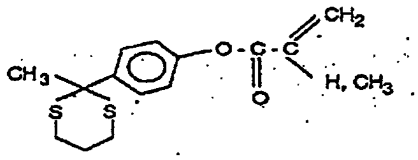

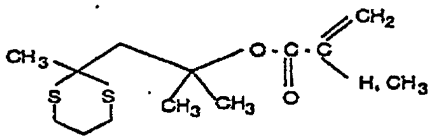

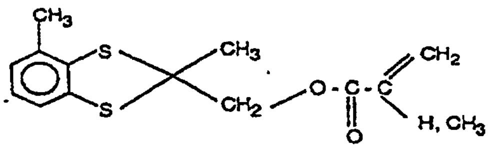

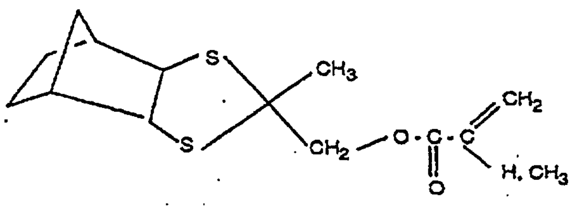

und

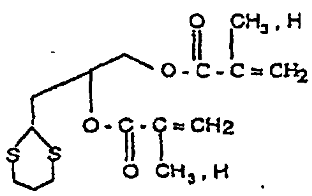

**4.** Verbindungen, die sich als Zwischenstufen für die Synthese monomerer Verbindungen gemäß Anspruch 1 eignen, **dadurch gekennzeichnet, dass** sie der Formel

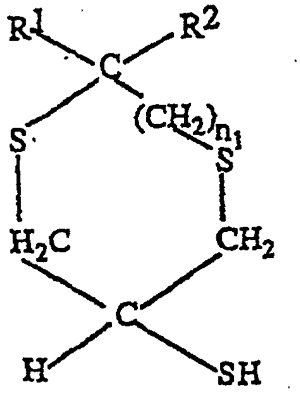

entsprechen, wobei $R^1$, $R^2$ und $n_1$ wie in Anspruch 1 definiert sind.

**5.** Verbindungen gemäß Anspruch 4, **dadurch gekennzeichnet, dass** sie aus Verbindungen der Formeln

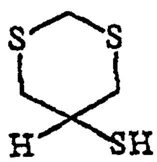 und 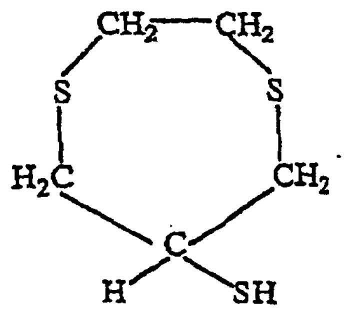

ausgewählt sind.

**6.** Polymerisierbare Zusammensetzung, **dadurch gekennzeichnet, dass** sie wenigstens ein Monomer des Typs Mono(thio)(meth)acrylat oder Di-(meth)acrylat umfasst, das einen fünf- bis achtgliedrigen Heterocyclus trägt, der aus C-, H- und S-Atomen aufgebaut ist und 2 oder 3 intracyclische Schwefelatome trägt, wobei der Heterocyclus gegebenenfalls mit einem substituierten oder unsubstituierten aromatischen oder polycyclanischen $C_5$-$C_8$-Cyclus, vorzugsweise einem $C_6$-$C_7$-Cyclus, kondensiert ist.

**7.** Polymerisierbare Zusammensetzung gemäß Anspruch 6, **dadurch gekennzeichnet, dass** der Heterocyclus sechsgliedrig ist.

**8.** Polymerisierbare Zusammensetzung gemäß Anspruch 6 oder 7, **dadurch gekennzeichnet, dass** das Monomer eine Molmasse zwischen 150 und 400, vorzugsweise zwischen 150 und 350 und besonders bevorzugt zwischen 200 und 300 hat.

**9.** Polymerisierbare Zusammensetzung gemäß einem der Ansprüche 6 bis 8, **dadurch gekennzeichnet, dass** der Heterocyclus zwei intracyclische Schwefelatome auf Position 1-3 oder 1-4 des Heterocyclus umfasst.

**10.** Polymerisierbare Zusammensetzung gemäß einem der Ansprüche 6 bis 8, **dadurch gekennzeichnet, dass** der Heterocyclus drei intracyclische Schwefelatome umfasst.

**11.** Polymerisierbare Zusammensetzung gemäß einem der Ansprüche 6 bis 9, **dadurch gekennzeichnet, dass** das Monomer vom Typ Thio(meth)acrylat ist.

**12.** Polymerisierbare Zusammensetzung, **dadurch gekennzeichnet, dass** sie wenigstens ein Monomer gemäß Anspruch 1, 2 oder 3 umfasst.

**13.** Polymerisierbare Zusammensetzung gemäß einem der Ansprüche 6 bis 12, **dadurch gekennzeichnet, dass** sie außerdem ein oder mehrere Comonomere umfasst, die von den Monomeren der Formel (A) verschieden sind.

**14.** Polymerisierbare Zusammensetzung gemäß Anspruch 13, **dadurch gekennzeichnet, dass** das oder die Comonomere aus vinylischen, acrylischen, methacrylischen Monomeren und Allylcarbonaten linearer oder verzweigter aliphatischer oder aromatischer flüssiger Polyole ausgewählt sind.

**15.** Polymerisierbare Zusammensetzung gemäß einem der Ansprüche 6 bis 14, **dadurch gekennzeichnet, dass** sie außerdem einen Polymerisationsstarter umfasst.

**16.** Polymerisierbare Zusammensetzung gemäß Anspruch 15, **dadurch gekennzeichnet, dass** der Polymerisationsstarter ein Photopolymerisationsstarter ist.

**17.** Polymerisierbare Zusammensetzung gemäß Anspruch 16, **dadurch gekennzeichnet, dass** der Photopolymerisationsstarter aus 2,4,6-Trimethylbenzoyldiphenylphosphinoxid, 1-Hydroxycyclohexylphenylketon, 2,2-Dimethoxy-1,2-diphenylethan-1-on, Alkylbenzoinethern und Gemischen davon ausgewählt ist.

**18.** Transparente polymere Zusammensetzung, die aus der Polymerisation einer der polymerisierbaren Zusammensetzungen gemäß den Ansprüchen 6 bis 17 stammt.

**19.** Transparente polymere Zusammensetzung, die aus der Photopolymerisation der polymerisierbaren Zusammensetzung gemäß Anspruch 16 oder 17 stammt.

**20.** Optischer oder ophthalmischer Artikel, der durch Formpressen und Polymerisation einer Zusammensetzung gemäß einem der Ansprüche 6 bis 17 erhalten wird.

**21.** Ophthalmischer Artikel gemäß Anspruch 20, **dadurch gekennzeichnet, dass** der Artikel eine ophthalmische Linse ist.

**Claims**

**1.** Monomer component, **characterized in that** it has the formula:

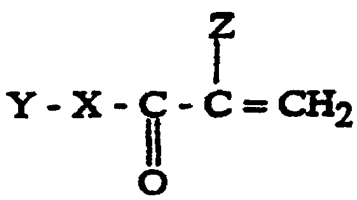

(A)

wherein Z represents H or $CH_3$ and X represents O or S, and

- when X represents S, Y is a radical of formula:

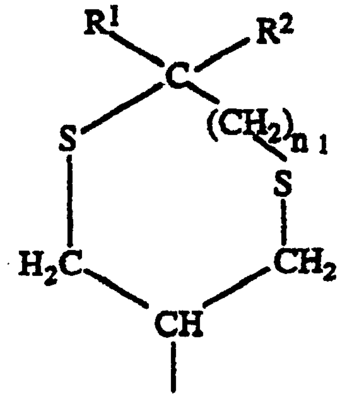

(a)

wherein $R^1$ and $R^2$ are selected from H, alkyl radicals, preferably $C_1$-$C_4$ alkyl radicals and more preferably a $CH_3$ radical, or alternatively $R^1$ and $R^2$ together are a $-(CH_2)_5-$ radical and $n_1$ is an integer from 0 to 2 inclusive, and

- when X represents O, Y is the radical (a) defined above or a radical selected from the radicals of formulae:

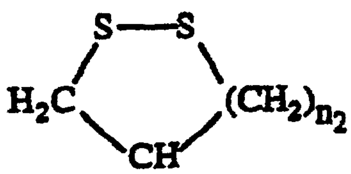

(b),

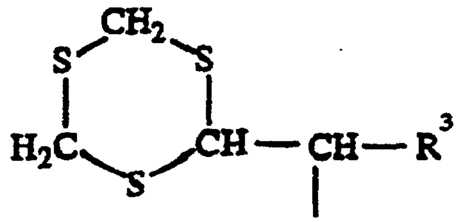

(c)

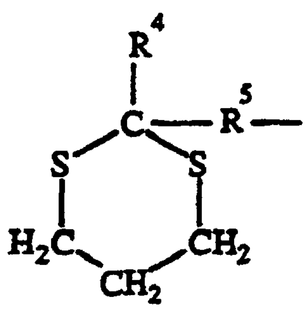

(d) and

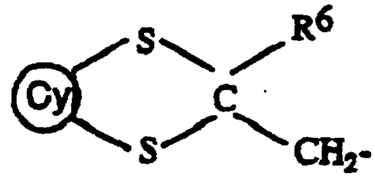

(e)

wherein $n_2$ is an integer equal to 1 or 2, $R^3$ represents H or an alkyl group, preferably a $C_1$-$C_4$ alkyl group and more preferably a methyl group, $R^4$ represents H, an alkyl group or an aryl group; the alkyl group preferably being a $C_1$-$C_4$ alkyl group and more preferably a methyl or ethyl group and the aryl group preferably being a $C_6$-$C_{12}$ aryl group and more preferably a phenyl group, and $R^5$ is a divalent radical selected from the groups of the following formulae:

(I)

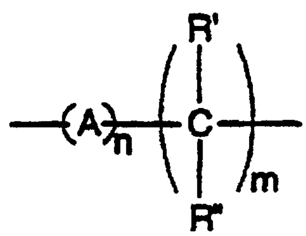

wherein:

A is an aryl group, preferably a $C_6$-$C_{12}$ aryl group and more preferably a phenyl group, or an alkyl group, preferably a $C_1$-$C_6$ alkyl group,
R' and R'' are, independently of each other, H, an alkyl group, preferably a $C_1$-$C_6$ alkyl group, an aryl group preferably a phenyl group, or R' or R'' can be a group

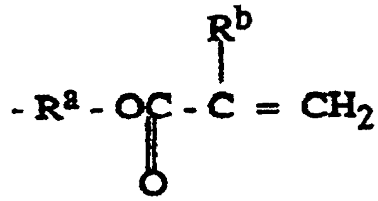

wherein $R^a$ is an alkylene group, preferably a $C_1$-$C_6$ alkylene group, in particular a $-CH_2-$ group, and $R^b$ is H or $CH_3$,
n has the values 0 or 1 and 0 ≤ m (integer ≤ 4), and

(II) 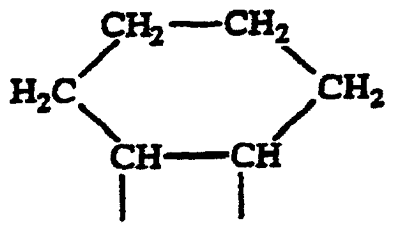

and
$R^6$ is H or a methyl group, and
Cy is a substituted or unsubstituted aryl ring, preferably a phenyl, tolyl or norbornyl ring.

2. Monofunctional monomer component of mono-(thio)(meth)acrylate type, according to claim 1, **characterized in that** $R^5$ is a divalent radical selected from:

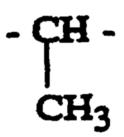 , 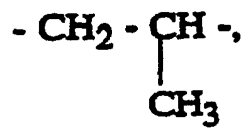 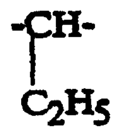

and

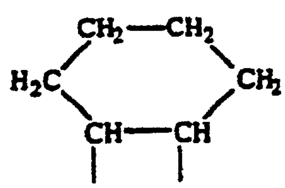

and Cy is a phenyl, tolyl or norbornyl group.

3. Monomer component according to claim 1, **characterized in that** it is selected from the compounds of formulae:

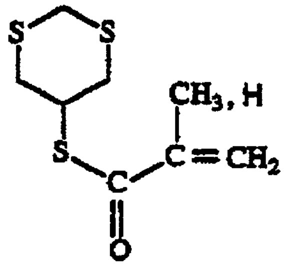 ,

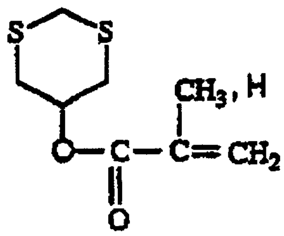 , 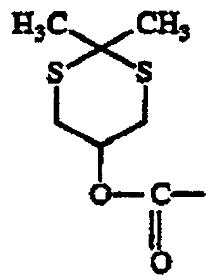 ,

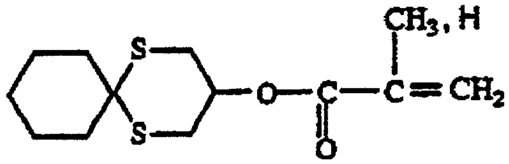 , 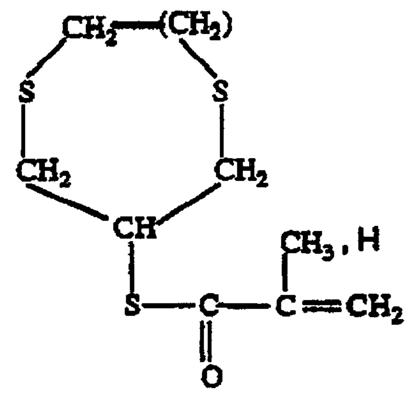 ,

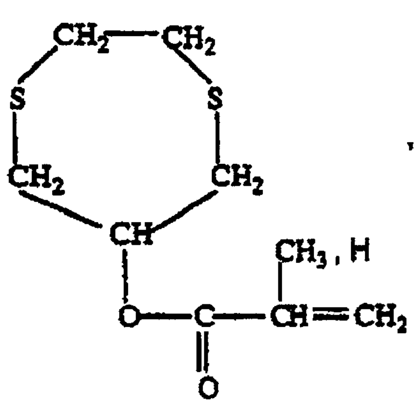 , 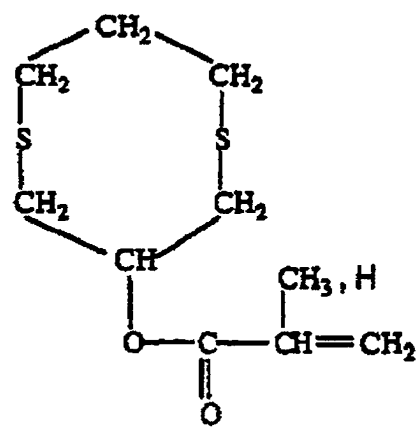 ,

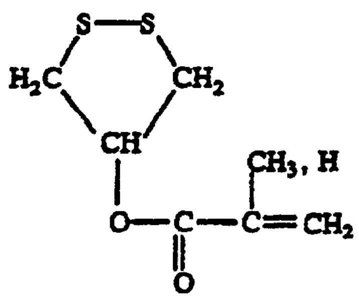
,
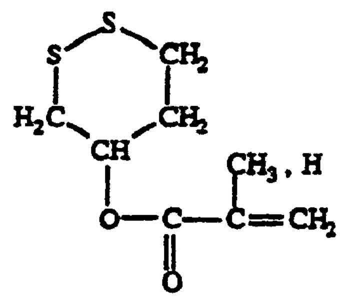
,

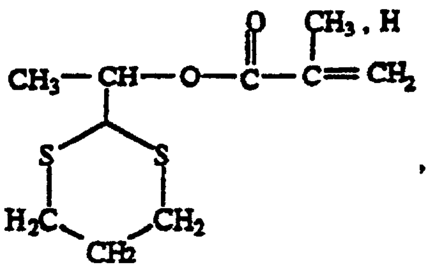
,
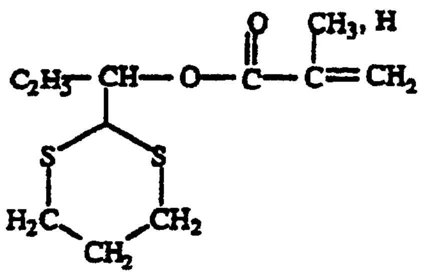
,

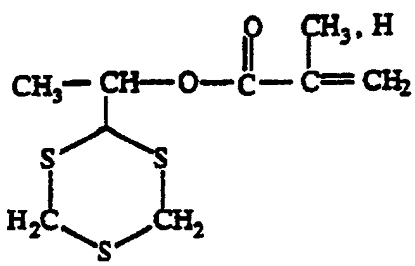
,
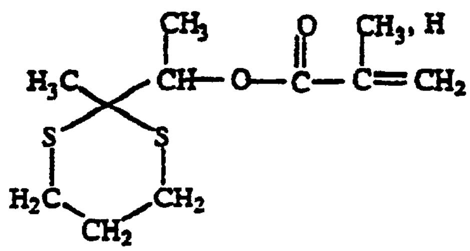
,

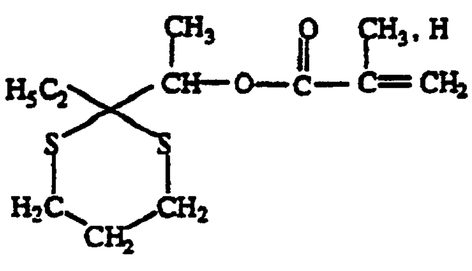
,
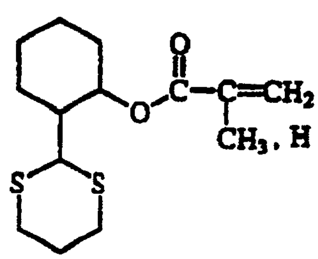
,

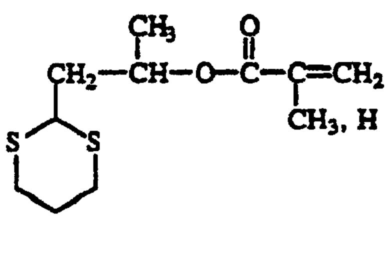

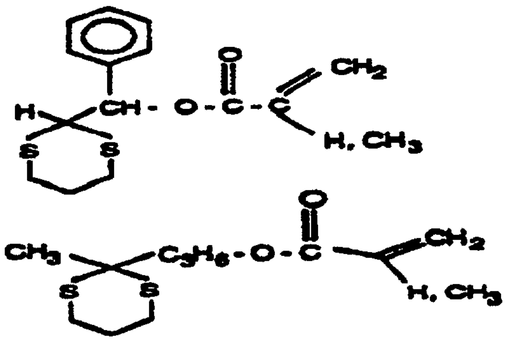

.

.

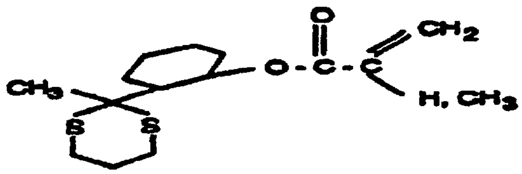

.

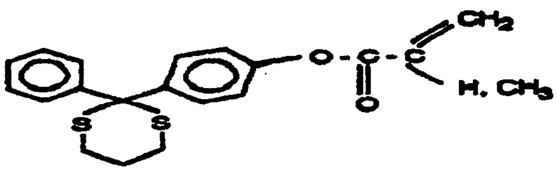

.

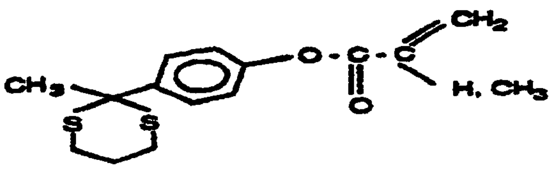

.

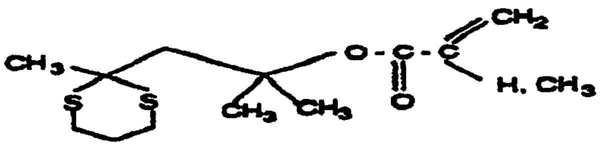

.

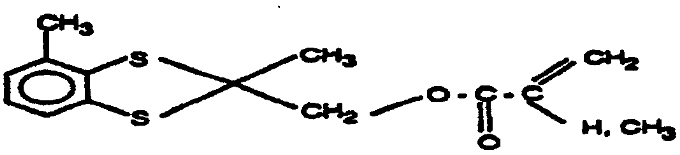

.

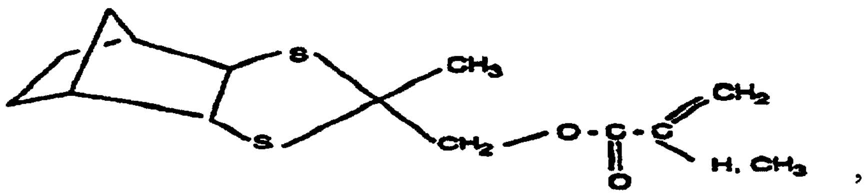

and

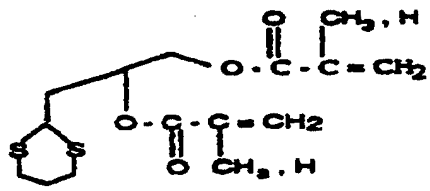

**4.** Components which are useful as intermediates for the synthesis of the monomer components according to claim 1, **characterized in that** they have the formula:

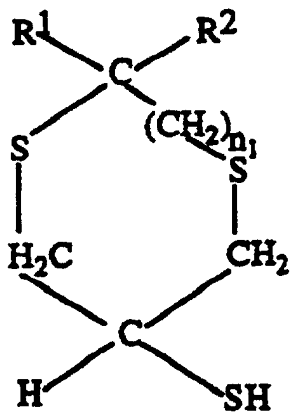

wherein $R^1$, $R^2$ and $n_1$ are defined as in claim 1.

**5.** Components according to claim 4, **characterized in that** they are selected from the compounds of formulae:

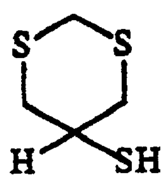

and

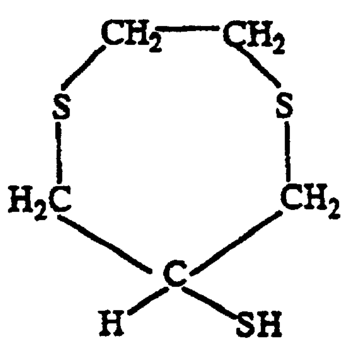

**6.** Polymerizable composition, **characterized in that** it comprises at least one monomer of mono(thio)(meth)acrylate or di(meth)acrylate type having a 5- to 8-membered heterocycle consisting of C, H and S atoms and containing 2

or 3 endocyclic sulphur atoms, the heterocycle optionally being fused to a substituted or unsubstituted $C_5$-$C_8$ aromatic or polycyclanic ring, preferably a $C_6$-$C_7$ ring.

**7.** Polymerizable composition according to claim 6, **characterized in that** the heterocycle is a 6-membered heterocycle.

**8.** Polymerizable composition according to claim 6 or 7, **characterized in that** the monomer has a molecular weight between 150 and 400, preferably between 150 and 350 and more preferably between 200 and 300.

**9.** Polymerizable composition according to any one of claims 6 to 8, **characterized in that** the heterocycle comprises two endocyclic sulphur atoms in positions 1-3 or 1-4 of the heterocycle.

**10.** Polymerizable composition according to any one of claims 6 to 8, **characterized in that** the heterocycle comprises three endocyclic sulphur atoms.

**11.** Polymerizable composition according to any one of claims 6 to 9, **characterized in that** the monomer is a thio (meth)acrylate monomer type.

**12.** Polymerizable composition, **characterized in that** it comprises at least one monomer according to claim 1, 2 or 3.

**13.** Polymerizable composition according to any one of claims 6 to 12, **characterized in that** it also comprises one or more comonomers other than the monomers of formula (A).

**14.** Polymerizable composition according to claim 13, **characterized in that** the comonomer(s) is(are) selected from vinyl, acrylic or methacrylic monomers and allylcarbonates of linear or branched, aliphatic or aromatic liquid polyols.

**15.** Polymerizable composition according to any one of claims 6 to 14, **characterized in that** it also comprises a polymerization initiator.

**16.** Polymerizable composition according to claim 15, **characterized in that** the polymerization initiator is a photopolymerization initiator.

**17.** Polymerizable composition according to claim 16, **characterized in that** the photoinitiator is selected from 2,4,6-trimethylbenzoyldiphenylphosphine oxide, 1-hydroxycyclohexyl phenyl ketone, 2,2-dimethoxy-1,2-diphenyl-1-ethanone and alkylbenzoin ethers, and mixtures thereof.

**18.** Transparent polymer composition resulting from the polymerization of any one of the polymerizable compositions according to claims 6 to 17.

**19.** Transparent polymer composition resulting from the photopolymerization of the polymerizable composition according to claim 16 or 17.

**20.** Optical or ophthalmic article obtained by moulding and polymerization of a composition according to any one of claims 6 to 17.

**21.** Ophthalmic article according to claim 20, **characterized in that** the article is an ophthalmic lens.